# EUROPEAN PATENT APPLICATION

(11) **EP 4 600 272 A1**
(43) Date of publication of application: **13.08.2025**
(21) Application number: 23871010.7
(22) Date of filing: 28.09.2023
(51) Int. Cl.: C07K 19/00, C07K 16/28, C07K 14/715, A61K 39/395, A61K 38/21, A61P 31/20

(54) **TREATMENT OF CHRONIC HEPATITIS B BASED ON INTERFERON SYNERGISTIC IMMUNE CHECKPOINT BLOCKING ANTIBODY**

(30) Priority: 29.09.2022 CN 202211201222
(71) Applicant: ImmuneLogic Therapeutics, Inc., Beijing 102206 (CN)
(72) Inventor: PENG, Hua, Beijing 100101 (CN); FU, Yangxin, Beijing 100101 (CN); MENG, Chaoyang, Beijing 100101 (CN)
(74) Representative: Nederlandsch Octrooibureau
(86) International application number: PCT/CN2023/122466
(87) International publication number: WO 2024/067785

(57) **Abstract**

Provided is use for the treatment of chronic hepatitis B based on the synergy of interferon and immune checkpoint blocking antibody. Specifically, provided is a fusion protein, comprising a PDL1 binding polypeptide and an interferon IFN which are operably linked. The fusion protein is a heterodimeric protein or a homodimeric protein. The fusion protein can achieve the anti-viral and immunomodulatory effects of IFN locally in the liver by means of intravenous injection, thereby improving the effectiveness, and reducing the side effects.

## Description

### TECHNICAL FIELD

The present invention belongs to the field of genetic engineering and biomedical technology, and specifically relates to the application of a fusion protein of interferon (IFN) and anti-PDL1 antibody in the treatment of chronic hepatitis B, as well as in the prevention of viral infections and in the treatment of chronic viral infections and latent viral recurrent infections *in vivo.*

### BACKGROUND

Hepatitis B virus (HBV) is a DNA virus belonging to the hepadnaviridae. Intact HBV is granular in shape, has a diameter of 42 nm, and consists of two parts of a envelope and a nucleocapsid. The envelope contains the small (S), medium (M), and large (L) envelope proteins (HBsAgs), as well as glycoproteins and cellular fats. The core particle contains a core protein (HBcAg), a partially cyclic double-stranded DNA of 3.2 kb, and a polymerase. HBVs adhere to the surface of hepatocytes, and enter into the cells through the cellular endocytosis mediated by the binding of the preS1 region of the large envelope protein (L- HBsAg) and the viral receptor of sodium-taurocholate cotransporting polypeptide (NTCP), thereby establishing the infection. Only humans, chimpanzees, and tree shrews are susceptible to HBVs. HBV infection can cause acute or chronic hepatitis, liver fibrosis, cirrhosis and liver cancer. There are currently approximately 240 million chronic hepatitis B patients worldwide, and approximately 1 million people die each year from cirrhosis or liver cancer caused by HBV. Whether HBV infection will develop into chronic hepatitis B (CHB) depends on the age and the immune system state of the host. About 80-90% of people infected with HBV in infancy will develop into chronic hepatitis B; 30-50% of people infected with HBV before the age of six will develop into chronic hepatitis B; and fewer than 5% of people infected with HBV in adulthood will develop into chronic hepatitis B.

HBV infection is a global health problem. Elimination of viral infection and reduction of complications (including liver fibrosis, cirrhosis and liver cancer) caused by CHB, are the main aims of the current treatment of CHB. Drugs that have been approved and marketed for the treatment of CHB include type I interferons (IFNs) and five nucleos(t)ide analogs (NAs). Type I interferons act as a bridge between innate immunity and adaptive immunity by promoting the maturation of dendritic cells (DCs), processing and presenting the antigens to activate T cells. IFNs enable a small proportion of CHB patients to suppress HBV replication after treatment for a limited period of time (usually 48 weeks) through their modulated immune activity and/or through one or more antiviral interferon-stimulated gene (ISG) products. As the type I interferon receptors (IFNARs) are widely expressed in tissues, they can induce severe side effects in patients with an increasing dose and a prolonged duration of administration, including the influenza-like symptoms (fever, headache, etc.), nausea, fatigue, leukopenia, anaemia, thrombocytopenia, etc., which seriously affects the life quality of patients. Most patients discontinue the drugs due to intolerance of their side effects. In addition, type I interferons can upregulate the expression of the immunosuppressive molecule-programmed death ligand 1 (PDL1)-to suppress the antiviral immune response and lead to a reduced therapeutic efficacy. How to overcome the side effects and the immunosuppression caused by the systemic administration of type I interferons is an important issue to be addressed. Nucleos(t)ide analogs inhibit the viral reverse transcriptase, thereby inhibiting the replication of HBV. Compared with type I interferons, the nucleos(t)ide analogs have the advantages that they can be administered orally, have a higher safety, and are able to significantly reduce the level of HBV-DNA in serum, but they rarely achieve the serological transformation of HBsAg (an indicator of functional cure) and may require a lifelong administration. Due to the persistence of superhelical covalently closed circular DNA (cccDNA) molecules in liver during the HBV infection, there is no effective method to silence or eliminate cccDNAs, so that CHB cannot be completely cured. The current consensus for the treatment of CHB is that a functional cure (*i.e.,* a serum HBsAg clearance accompanied by the production of anti-HBsAg antibodies, and a serum HBV-DNA level below the detection limit), and the functional cure is an ideal endpoint of the current treatment of CHB that may be achieved.

Programmed cell death protein 1 (PD1) is a key immune checkpoint molecule that inhibits the TCR signaling activation in T cells, thereby suppressing the immune response. The ligand of PD1, PDL1, is generally up-regulated in livers of patients with CHB, and at the same time, T cells overexpress the inhibitory receptor PD1 in CHB patients. The binding of PD1 and PDL1 reduces the T-cell functions, resulting in no effective immune response in liver, and consequently no inhibition or clearance of HBV. Currently, PD1/PDL1 blocking therapy has been applied to the treatment of a variety of tumors, and the studies of PD1/PDL1 blocking therapy in the treatment of HBV have been reported. The PD1 antibody (nivolumab) has been used to treat the HBeAg-negative CHB patients, and the results showed that the therapy restored the HBV-specific immune response to a certain extent, but the percentage of functional cure was not high (only 1 out of 10 cases achieved the serum HBsAg clearance). How to improve the percentage of functional cure of CHB by using the PD1/PDL1 blocking therapy needs to be further investigated.

Since the successful development of prophylactic hepatitis B vaccine in 1982, it has been widely used worldwide with a good protective effect. Vaccination with the prophylactic hepatitis B vaccine has greatly reduced the rate of HBV infection, but it does not produce neutralizing antibodies in the patients with chronic hepatitis B and the virus carriers. In patients with chronic hepatitis B whose immune systems are already tolerant to HBV, it is more difficult to induce a specific immune response. Therefore, the research on the therapeutic chronic hepatitis B vaccines has a long way to go. So far, the therapeutic hepatitis B vaccine research is mainly on a DNA vaccine, a DC vaccine and a protein vaccine, etc. DNA vaccine is a nucleic acid sequence encoding antigen by using a plasmid or a modified virus as a vector. DNA vaccine can induce the strong continuous humoral immunity and cellular immunity by virtue of the continuous antigen synthesis *in vivo* and its own immunomodulatory ability. However, the DNA vaccine has not been shown to have the effective therapeutic effects in the HBV transgenic mice and the non-human primate models, as well as in the clinical results. Antigen-carrying DC cells were used as a vaccine to promote the specific immune responses. The DC vaccine was able to elicit a specific cytotoxic T lymphocyte (CTL) response in the HBV transgenic mouse model. But there was no significant reduction in the level of either HBV-associated RNA or DNA in liver, suggesting that the number of CTLs elicited in the transgenic mice was insufficient and dysfunctional. By using the HBV prophylactic vaccines (with HBsAg as an antigen and Alum as an adjuvant) as the basis, increasing the dose and number of vaccine immunizations, adding the preS1/preS2 antigen, or altering the structural morphology of the recombinant proteins, the researchers have found a decrease in HBV-DNA, a return of ALT level to normal, and a serological transformation of HBeAg in a small number of patients in the clinical trials, but none of them were able to clear HBV-DNA and induce a serological transformation of HBsAg.

### SUMMARY OF THE INVENTION

For the currently approved drugs marketed for the treatment of CHB patients, neither the type I interferons (IFNs) nor the nucleos(t)ide analogs (NAs) can achieve a functional cure. IFNs have a low response rate to the systemic administration and a great side effect. In order to overcome the limitations and deficiencies of the existing IFNs for the treatment of CHB patient and in consideration of the high expression of PDL1 in livers of CHB patients, the present invention provides a fusion protein of an anti-PDL1 antibody and an IFN (anti-PDL1-IFN), which targets the IFN to the liver using the anti-PDL1 antibody to achieve the anti-viral and immunomodulatory effects of the IFN locally, as well as improve the effectiveness and reduce the side-effects. On the one hand, the anti-PDL1-IFN fusion protein targets the IFN to the liver to exert the inhibitory effect of HBV. At the same time, the IFN can be targeted to the DC cells in liver to improve the function of DC cells and thereby promote the function of HBV-specific T cells, as well as play the blocking role of an immune checkpoint, overcome the PDL1-mediated immunosuppression, and achieve the immunomodulatory effect. IFN-induced upregulation of PDL1 in liver is conducive to a more effective liver targeting of the anti-PDL1-IFN fusion protein. In the HBV carrier mouse experimental model, a more significant accumulation of the anti-PDL1-IFN fusion protein in liver was observed, while the therapeutic efficacy of the anti-PDL1-IFN fusion protein was significantly higher than those of the non-targeted IFN or anti-PDL1 antibody as well as the physical mixture of the non-targeted IFN and anti-PDL1 antibody. Using the anti-viral effect of anti-PDL1-IFN fusion protein to reduce the levels of HBsAg and HBV-DNA in liver and peripheral blood, as well as its immunomodulatory effect, to achieve the formation of a "window period" to break the immune tolerance, and the combined active immunization with the vaccine, is capable of inducing the HBV-specific T-cell and B-cell responses in the hosts, thereby achieving a functional cure for chronic hepatitis B.

The present invention also relates to the application of a mixture of anti-PDL1-IFN with a HBV antigen (or other viral antigen and a tumor antigen), or a homo- or hetero-dimeric fusion protein, or a homo- or hetero-multimeric fusion protein composed of anti-PDL1-IFN and HBV antigen (or other viral antigen and a tumor antigen) as a prophylactic or therapeutic vaccine.

The object of the present invention is achieved by the following technical solutions:
The present invention provides a fusion protein comprising a PDL1-binding polypeptide and an interferon (IFN) which are operably linked.

In specific embodiments of the present invention, said fusion protein is a heterodimeric protein or a homodimeric protein.

The heterodimer of the fusion proteins described in the present application (also referred to as an anti-PDL1-IFN heterodimeric fusion protein) may for example include two forms:
1) in the first form, the PDL1-binding polypeptide is in the form of a Fab (or referred to as a heter Fab), said fusion protein comprises a first polypeptide, a second polypeptide and a third polypeptide, and each of said first polypeptide, second polypeptide and third polypeptide is different; wherein, said first polypeptide comprises an IFN and an immunoglobulin Fc region, with the IFN being located at the N-terminus or C-terminus of the Fc region; said second polypeptide comprises a Fab region of the heavy chain of the PDL1-binding polypeptide and an immunoglobulin Fc region, with the Fab region of the heavy chain of the PDL1-binding polypeptide being located at the N-terminus or C-terminus of the Fc region; and said third polypeptide is the light chain of the PDL1-binding polypeptide.
   Preferably, said first polypeptide comprises the amino acid sequence as set forth in SEQ ID NO.1, SEQ ID NO.19, SEQ ID NO.23 or SEQ ID NO.25, said second polypeptide comprises the amino acid sequence as set forth in SEQ ID NO.5 or SEQ ID NO.21, and said third polypeptide comprises the amino acid sequence as set forth in SEQ ID NO.3;
2) in the second form, the PDL1-binding polypeptide is in the form of a scFv (or referred to as a heter scFv), said fusion protein comprises a first polypeptide and a second polypeptide, and said first and second polypeptides are different; wherein, said first polypeptide comprises an IFN and an immunoglobulin Fc region, with the IFN being located at the N-terminus or C-terminus of the Fc region; and said second polypeptide comprises a PDL1-binding polypeptide in the form of a scFv and an immunoglobulin Fc region, with the PDL1-binding polypeptide in the form of a scFv being located at the N-terminus or C-terminus of the Fc region;

Preferably, said first polypeptide comprises the amino acid sequence as set forth in SEQ ID NO.1 or SEQ ID NO.23, and said second polypeptide comprises the amino acid sequence as set forth in SEQ ID NO.9.

The Fc region contained by said first polypeptide and the Fc region contained by said second polypeptide are derived from the same or different subtypes of immunoglobulins.

The homodimer of the fusion protein described in the present invention (also referred to as an anti-PDL1-IFN homodimeric protein) may for example include two forms:
1) in the first form, the PDL1-binding polypeptide is in the form of a Fab (or referred to as a homo Fab), and said fusion protein comprises four polypeptides, wherein the first polypeptide is identical to the second polypeptide, and the third polypeptide is identical to the fourth polypeptide; the first and second polypeptides sequentially comprise, from the N-terminus to the C-terminus, an IFN, a Fab region of the heavy chain of the PDL1-binding polypeptide, and an immunoglobulin Fc region (optionally, said IFN, said Fab region of the heavy chain of the PDL1-binding polypeptide and said immunoglobulin Fc region may also be combined and arranged in any order), preferably comprise the amino acid sequence as set forth in SEQ ID NO. 7; the third polypeptide and the fourth polypeptide are the light chain of the PDL1-binding polypeptide, preferably comprise the amino acid sequence as set forth in SEQ ID NO. 3.
2) in the second form, the PDL1-binding polypeptide is in the form of a scFv (or referred to as a homo scFv), said fusion polypeptide comprises a first polypeptide and a second polypeptide, and said first and second polypeptides are identical. Said first and second polypeptides sequentially comprise, from the N-terminus to the C-terminus, an IFN, a PDL1-binding polypeptide in the form of a scFv and an immunoglobulin Fc region (optionally, said IFN, said PDL1-binding polypeptide in the form of a scFv and said immunoglobulin Fc region can also be combined and arranged in any order), preferably comprise the amino acid sequences as set forth in SEQ ID NO. 11.

In specific embodiments of the present invention, said interferon (IFN) can be selected from a type I interferon and a type I interferon mutant, preferably mouse IFNα4 (*e.g.,* the amino acids at positions 1-162 of SEQ ID NO. 1 or SEQ ID NO. 19) and human IFNα2 (*e.g.,* the amino acids at positions 1-165 of SEQ ID NO. 23 or SEQ ID NO. 25); said IFN can be of human or murine origin.

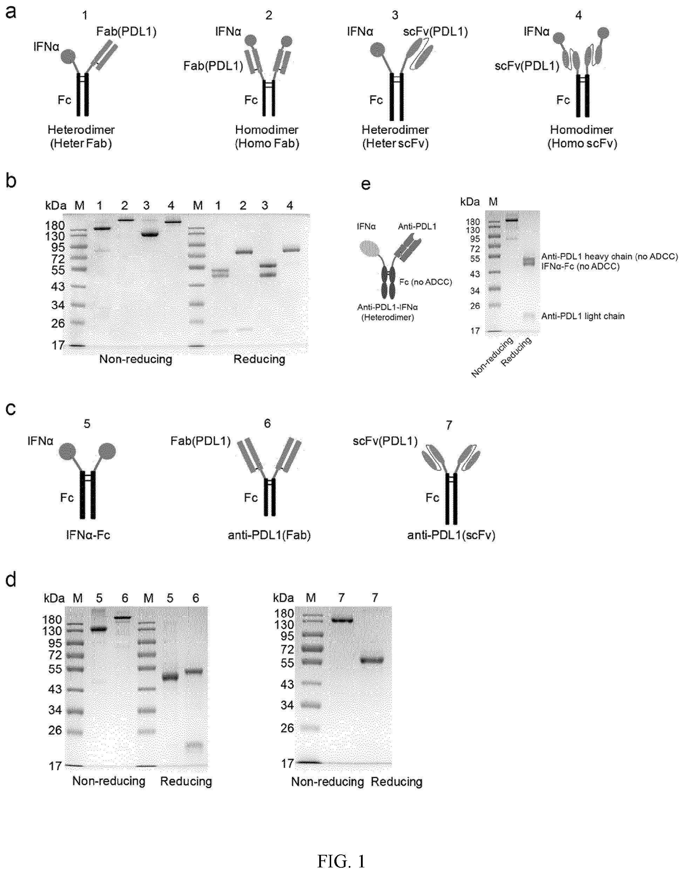

In specific embodiments of the present invention, said immunoglobulin Fc region can be selected from the amino acid sequence of the constant region of IgG1, IgG2, IgG3 or IgG4, preferably IgG1. IgG1 has a strong ability to induce the antibody-dependent cell-mediated cytotoxicity (ADCC) and the complement dependent cytotoxicity (CDC) effects and a long serum half-life, and is the most common antibody subtype of antibody drugs. The Fc region can also be mutated to form a Fc region in the no ADCC form. Said no ADCC form of the Fc region refers to the mutation of three amino acids in the Fc fragment of human IgG1, namely L234A, L235A, and P329G (referred to as a LALA-PG mutant), respectively. This mutant completely loses its ability to bind the Fcγ receptor (FcγR), thereby reducing the cytotoxicity (ADCC). The mutation of the Fc region to reduce the cytotoxicity is a technology known in the art, see for example Tilman Schlothauer et al., Novel human IgG1 and IgG4 Fc-engineered antibodies with completely abolished immune effector functions, Protein Engineering, Design & Selection, 2016, vol. 29, no. 10, pp. 457-466. In embodiments of the present invention, the Fc region of the fusion proteins in the four forms as set forth in Fig. 1a may be replaced with a mutated Fc region *(i.e.,* in the no ADCC form) which has a similar effect to that of the wild type.

In specific embodiments of the present invention, said PDL1-binding polypeptide can be selected from an anti-PDL1 antibody (an intact antibody), a single-chain variable fragment (scFv), a Fab fragment, a F(ab')₂ fragment; and said anti-PDL1 antibody is preferably selected from: Tecentriq, Bavencio, Imfinzi, KN035, CS1001, KL-A167, SHR-1316 or YW243.55.S70; more preferably a PDL1-binding Fab fragment.

Specifically, the present invention provides the following technical solutions:
1. A fusion protein comprising a PDL1-binding polypeptide and an interferon (IFN) which are operably linked.
2. The fusion protein according to item 1, which is a heterodimeric protein or a homodimeric protein.
3. The fusion protein according to item 1 which is a heterodimeric protein and said PDL1-binding polypeptide is in the form of a Fab, wherein:
   optionally, said fusion protein comprises a first polypeptide, a second polypeptide and a third polypeptide; wherein, said first polypeptide comprises an IFN and an immunoglobulin Fc region, and the IFN is located at the N-terminus or the C-terminus of the Fc region; said second polypeptide comprises a Fab region of the heavy chain of the PDL1-binding polypeptide and an immunoglobulin Fc region, and said Fab region of the heavy chain of the PDL1-binding polypeptide is located at the N-terminus or the C-terminus of the Fc region; and said third polypeptide is the light chain of the PDL1-binding polypeptide;
   preferably, said first polypeptide comprises or consists of the amino acid sequence as set forth in SEQ ID NO.1 or SEQ ID NO.19 or SEQ ID NO.23 or SEQ ID NO.25, said second polypeptide comprises or consists of the amino acid sequence as set forth in SEQ ID NO.5 or SEQ ID NO.21, and said third polypeptide comprises or consists of the amino acid sequence as set forth in SEQ ID NO.3;
   or, said fusion protein is a heterodimeric protein and said PDL1-binding polypeptide is in the form of a scFv, wherein:
      optionally, said fusion protein comprises a first polypeptide and a second polypeptide; said first polypeptide comprises an IFN and an immunoglobulin Fc region, and the IFN is located at the N-terminus or C-terminus of the Fc region; and said second polypeptide comprises a PDL1-binding polypeptide in the form of a scFv and an immunoglobulin Fc region, and the PDL1-binding polypeptide in the form of a scFv is located at the N-terminus or C-terminus of the Fc region;
      preferably, said first polypeptide comprises or consists of the amino acid sequence as set forth in SEQ ID NO. 1 or SEQ ID NO. 23, and said second polypeptide comprises or consists of the amino acid sequence as set forth in SEQ ID NO. 9.
4. The fusion protein according to item 1, wherein said fusion protein is a homodimeric protein and said PDL1-binding polypeptide is in the form of a Fab; wherein:
   optionally, said fusion protein comprises a first polypeptide and a second polypeptide which are identical, and a third polypeptide and a fourth polypeptide which are identical; wherein, said first polypeptide and second polypeptide comprise an IFN, a Fab region of the heavy chain of the PDL1-binding polypeptide, and an immunoglobulin Fc region, respectively, preferably comprise or consist of the amino acid sequence as set forth in SEQ ID NO. 7; and said third polypeptide and fourth polypeptide are respectively the light chain of the PDL1-binding polypeptide, preferably comprise or consist of the amino acid sequence as set forth in SEQ ID NO.3;
   or, said fusion protein is a homodimeric protein and said PDL1-binding polypeptide is in the form of a scFv; wherein:
      optionally, said fusion protein comprises a first polypeptide and a second polypeptide both of which are identical, wherein said first polypeptide and second polypeptide respectively comprise an IFN, a PDL1- binding polypeptide in the form of a scFv and an immunoglobulin Fc region, preferably comprise or consist of the amino acid sequence as set forth in SEQ ID NO. 11.
5. The fusion protein according to item 1, wherein said interferon (IFN) can be selected from a type I interferon and a type I interferon mutant, preferably mouse IFNα4 and human IFNα2; said IFN can be of human or murine origin.
6. The fusion protein according to item 3 or 4, wherein said immunoglobulin Fc region can be selected from the amino acid sequence of the constant region of IgG1, IgG2, IgG3 or IgG4 (preferably IgG1) or a mutant thereof .
7. The fusion protein according to item 1, wherein, said PDL1-binding polypeptide can be selected from an anti-PDL1 antibody or an antigen-binding fragment thereof, such as a single-chain variable fragment (scFv), a Fab fragment, a F(ab')₂ fragment; and said anti-PDL1 antibody is preferably selected from: Tecentriq, Bavencio, Imfinzi, KN035, CS1001, KL-A167, SHR-1316 or YW243.55.S70; more preferably, said PDL1-binding polypeptide is a Fab fragment of a PDL1 antibody.
8. Use of the fusion protein of any one of items 1-7 in the manufacture of a medicament for the treatment of chronic hepatitis B.
9. A pharmaceutical formulation or pharmaceutical composition comprising the fusion protein of any one of items 1-7 as an active ingredient.
10. A polynucleotide encoding the fusion protein according to any one of items 1-7.
11. A vector comprising the polynucleotide of item 10, wherein said vector is optionally a plasmid vector or a viral vector.
12. A cell comprising the polynucleotide of item 10 or the vector of item 11, wherein, said cell expresses the fusion protein of any one of items 1-7; preferably, said cell is selected from a non-human mammalian cell, preferably CHO and HEK293 cell.
13. A pharmaceutical composition or kit comprising the fusion protein of any one of items 1-7 and a vaccine; preferably said fusion protein and said vaccine are administered by sequential or simultaneous administration, and the administration method comprises an intravenous injection and a subcutaneous or intramuscular immunization, respectively, or a simultaneous subcutaneous or intramuscular immunization, etc.;
   optionally, the antigen in said vaccine can be an envelope protein of HBV (S-HBsAg, M-HBsAg, L-HBsAg) or a polypeptide thereof, a nucleocapsid protein (HBcAg) or a polypeptide thereof, a polymerase protein or a polypeptide thereof, a HBxAg protein or a polypeptide thereof, preferably an envelope protein of HBV or a polypeptide thereof; or the antigen in said vaccine can be a receptor-binding region antigen of SARS-CoV-2 or a HA1 antigen of influenza virus;
   optionally, said vaccine further comprises an adjuvant comprising an aluminium adjuvant, a liposome, or CpG, etc.
14. Use of the fusion protein of any one of items 1-7 or the combination of the fusion protein of any one of items 1-7 and a vaccine in the manufacture of a medicament or kit for the prevention of a viral infection (*e.g.*, an acute viral infection), for the treatment of a chronic or latent viral infection; optionally, said virus includes, but is not limited to, HBV, coronavirus, and influenza virus.

The fusion protein (anti-PDL1-IFN) of the present invention targets the IFN to the liver using the anti-PDL1 antibody to achieve the anti-viral and immunomodulatory effects of the IFN locally, as well as to improve the effectiveness and reduce the side effects. On the one hand, the anti-PDL1-IFN fusion protein targets the IFN to the liver to exert the inhibitory effect on HBV. At the same time, the IFN can be targeted to the DC cells in liver to improve the function of DC cells, thereby promoting the function of HBV-specific T cells. The fusion protein can also play the role of blocking an immune checkpoint, overcome the PDL1-mediated immunosuppression, and achieve the immunomodulatory effect. IFN-induced upregulation of PDL1 in liver is conducive to more effective targeting of the anti-PDL1-IFN fusion protein. Using the anti-viral effect of anti-PDL1-IFN fusion protein to reduce the levels of HBsAg and HBV-DNA in liver and peripheral blood, as well as its immunomodulatory effect, can achieve the formation of a "window period" to break the immune tolerance, and the combined active immunity with the vaccine, is capable of inducing the HBV-specific T-cell and B-cell responses in the hosts, or the antiviral neutralizing antibodies and T cell response can be produced by the immune induction using the mixture of the anti-PDL1-IFN fusion protein and a vaccine, thereby achieving a functional cure of chronic hepatitis B.

At the same time, the present invention can be used as an immunotherapeutic platform for the prevention of acute infections with coronaviruses and influenza viruses, and for the prevention and treatment of chronic infections with viruses such as HBV, HPV and EBV. The fusion protein of the present invention can be used as an adjuvant for a vaccine and mixed with the vaccine to function in the draining lymph nodes by the subcutaneous or intramuscular injection. Since the dendritic cells (DCs) in the lymph nodes are the PDL1 high-expressing cells, so that the fusion protein containing the anti-PD-L antibody and the IFN can more efficiently target the DCs, stimulate the activation of DCs, capture the antigens, and present the antigens, and enhance the immune response to the antigen in the vaccine, thereby significantly increasing the effectiveness of the vaccine, increasing the neutralizing antibody and T-cell responses against the antigens, clearing the viruses, and achieving the purpose of prevention or treatment against the corresponding viruses (but not limited to the specific viruses enumerated in the examples).

In summary, the fusion protein of the present invention can be administered by intravenous injection to achieve the anti-viral and immunomodulatory effects of the IFN in liver locally, improve the effectiveness and reduce the side effects. Using the anti-viral effect of the fusion protein of the present invention to reduce the levels of HBV virus and its antigens in liver and peripheral blood, as well as using the immunomodulatory effect of the PDL1-binding peptide to reduce the HBV immune tolerance, and then combining with the vaccine active immunization, is able to induce the HBV-specific T-cell and B-cell immune response in hosts. In addition, using the fusion protein of the present invention directly mixed with the vaccine to immunologically induce the production of anti-viral neutralizing antibodies and T-cell responses by the intramuscular injection, can achieve a functional cure of chronic viral infections, or the prevention of viral infections and the treatment of chronic viral infections and latent virus recurrent infection *in vivo.*

### TERMS AND DEFINITIONS

Unless otherwise specified, the terms and definitions used in the present application have the meanings customarily used in the art and are known to those skilled in the art.

As used in the present application, the term "administration" refers to a systemic and/or topical administration. The term "systemic administration" refers to a non-topical administration, whereby the substance administered may affect several organs or tissues in the whole body; or whereby the substance administered may travel through several organs or tissues in the whole body to reach the target site. It will be understood by those skilled in the art that said systemic administration encompasses various forms of administration including, but not limited to: a parenteral administration, an intravenous administration, an intramuscular administration, a subcutaneous administration, a transdermal administration, an oral administration, and the like. The term "topical administration" refers to an administration at or around a specific site. It will be understood by those skilled in the art that the topical administration encompasses various forms of administration, such as a direct injection at or around a specific site.

As used in the present application, the term "therapeutically effective amount" refers to the dose of the anti-PDL1-IFN fusion protein of the present invention required to achieve a therapeutic purpose (*e.g.,* a significant decrease of HBsAg or being negative for HBsAg in serum of a patient with Hepatitis B, or an improvement of the immune cell function). Said effective amount may be determined with respect to a particular purpose by practice or according to a conventional manner. In particular, said therapeutically effective amount may be the amount required to achieve: a decrease of HBsAg or being negative for HBsAg in serum of a patient with hepatitis B with or without the production of HBsAb, a decrease of HBeAg or being negative for HBeAg, a decrease of HBV-DNA or a decrease below the detection limit of HBV-DNA, a decrease of the hepatic necrotic inflammation or a recovery of the liver function, an activation of the immune cells, etc.

As used in the present application, the term "antibody" encompasses, for example: a monoclonal antibody, a polyclonal antibody, a single-chain variable fragment, an antibody fragment (which exhibits the desired biological or immunological activity). In the present application, the term "immunoglobulin" (Ig) is used interchangeably with the antibody specifically targeting the liver tissue.

As used in the present invention, the term "application" or "use" may denote both the applications for the purpose of treating diseases and the applications for non-therapeutic purposes, for example, a scientific research, etc.

In the present invention, said PDL1-binding polypeptide may be selected from: an anti-PDL1 intact antibody, a single-chain variable fragment (scFv), a Fab fragment, a F(ab')₂ fragment; wherein said anti-PDL1 intact antibody is a commercially available and conventional anti-PDL1 antibody, such as Tecentriq, Bavencio, Imfinzi, KN035, CS1001, KL-A167, SHR-1316 or YW243.55.S70.

### Beneficial effects of the present invention:

1. The anti-PDL1-IFN fusion protein provided by the present invention has heterodimeric or homodimeric structures, both of which have the effective anti-viral activity *in vitro.* The heterodimer has a superior liver targeting, a serum stability and a more effective HBV inhibition *in vivo.*
2. The present invention provides the anti-PDL1-IFN fusion protein with data showing that the anti-PDL1 antibody (the PDL1-binding polypeptide) can be used to specifically deliver the IFN to the liver tissue with fewer side effects. The present invention lays the foundation for the development of novel IFN-targeting drugs for the treatment of CHB.
3. The anti-PDL1-IFN fusion protein provided by the present invention may be combined with an active immunization of vaccine, wherein the combination of said fusion protein with a HBsAg/CpG vaccine or a commercial hepatitis B prophylactic vaccine can achieve a functional cure in some HBV carrier mice.
4. The anti-PDL1-IFN fusion protein provided by the present invention is an immune adjuvant platform that can be used for the prevention of various acute viral infections, the treatment of chronic or latent viral infections and virus-associated tumors.

### DESCRIPTION OF THE DRAWINGS

Figure 1. Construction of the anti-PDL1-IFN fusion proteins and the control proteins. (a) Schematic representation of the four anti-PDL1-IFN fusion proteins in the form of heterodimer or homodimer. Fab: antigen-binding fragment; and scFv: single-chain variable fragment. (b) Four anti-PDL1-IFN fusion proteins were expressed in 293F cells, purified and analyzed by the non-reducing and reducing SDS-PAGEs. M: molecular weight marker; 1: Fab(PDL1) heterodimer (Heter Fab); 2: Fab(PDL1) homodimer (Homo Fab); 3: scFv(PDL1) heterodimer (Heter scFv); and 4: scFv(PDL1) homodimer (Homo scFv). (c) Schematic representation of the 3 control fusion proteins IFNα-Fc, anti-PDL1(Fab) and anti-PDL1(scFv). (d) These 3 control fusion proteins were expressed in 293F cells, purified and analyzed by the non-reducing and reducing SDS-PAGEs. M: molecular weight marker; 5: IFNα-Fc; 6: anti-PDL1(Fab); and 7: anti-PDL1(scFv). (e) Schematic representation of anti-PDL1-IFN fusion protein wherein the Fc fragment is in the form of no ADCC, as well as SDS-PAGE result of the fusion protein after being expressed in 293F cells, purified and analyzed by the non-reducing and reducing SDS-PAGEs. M: molecular weight marker.

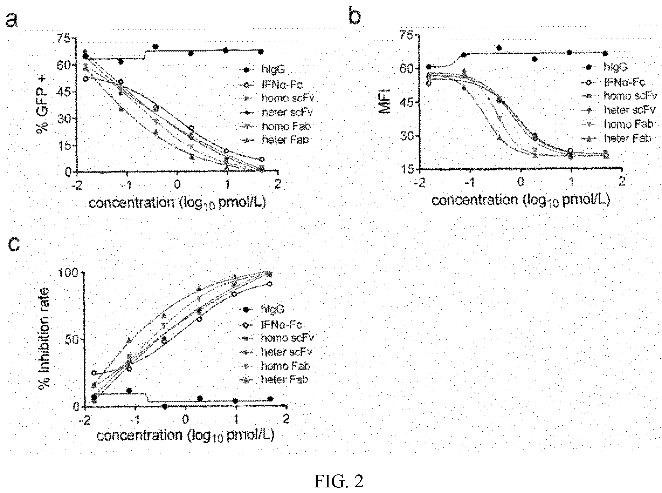

Figure 2. All four anti-PDL1-IFN fusion proteins maintain activities of the IFN in resisting viral infection and inhibiting viral replication. (a) The biological activity of the IFN in the four anti-PDL1-IFN fusion proteins was detected by an anti-viral infection bioassay. L929 cells (6 × 10⁴ cells/well) were incubated with each fusion protein (6 dilution gradients) in a 24-well plate for 12 hours. The cells were then infected with the vesicular stomatitis virus-green fluorescent protein (VSV-GFP) (4 × 10⁵ pfu/well) and incubated for an additional 30 hours. Then, the percentages of virus-infected cells (% GFP⁺) were detected by flow cytometry. (b) MFIs of the virus-infected cells (GFP⁺) were detected by flow cytometry. (c) Inhibition rates of the four anti-PDL1-IFN fusion proteins on the VSV-GFP virus-infected L929 cells.

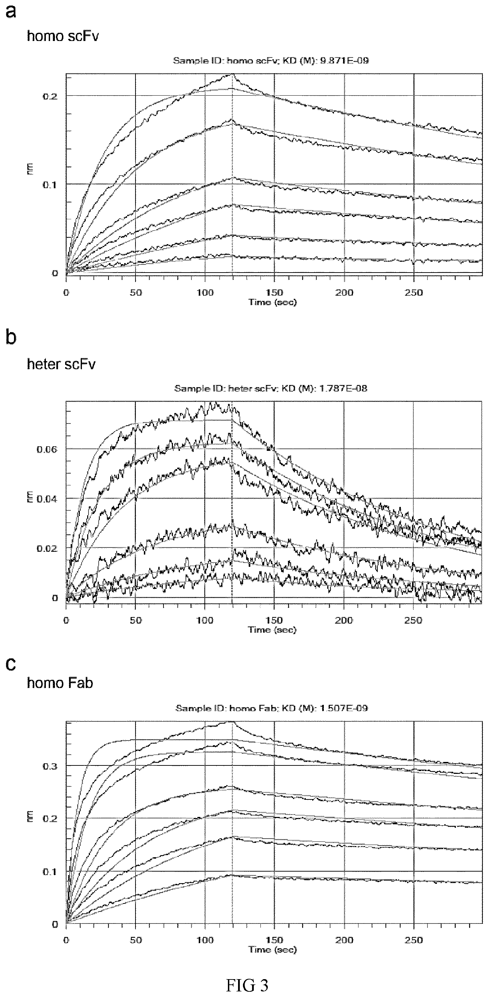

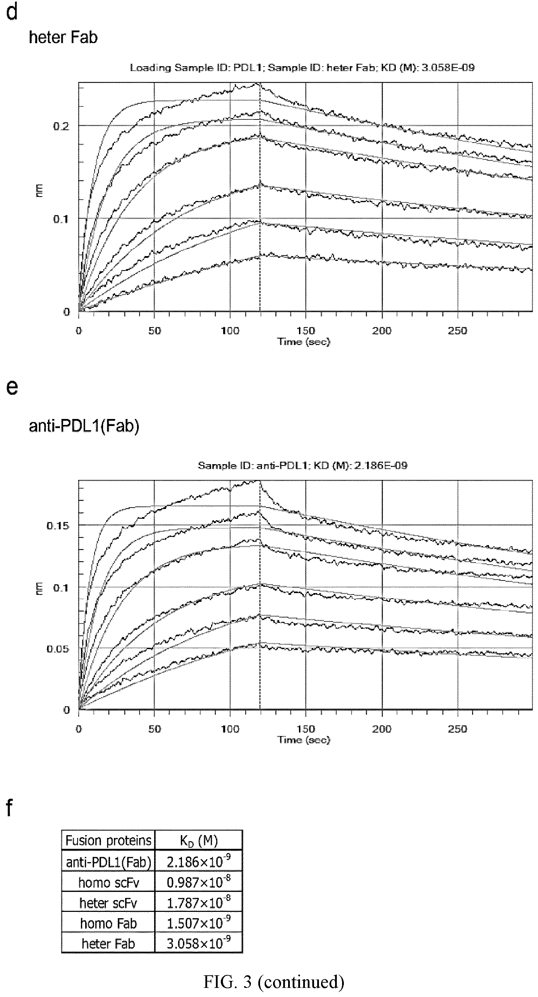

Figure 3. All four anti-PDL1-IFN fusion proteins maintain the affinities for PDL1. (a-e) The association and dissociation curves of the homo scFv fusion protein (a), the heter scFv fusion protein (b), the homo Fab fusion protein (c), the heter Fab fusion proteins (d), and anti-PDL1 (Fab) (e) with PDL1 were measured using the Biolayer Interferometry (BLI). (f) Affinity constants of the four anti-PDL1-IFN fusion proteins and anti-PDL1(Fab) with PDL1 were calculated.

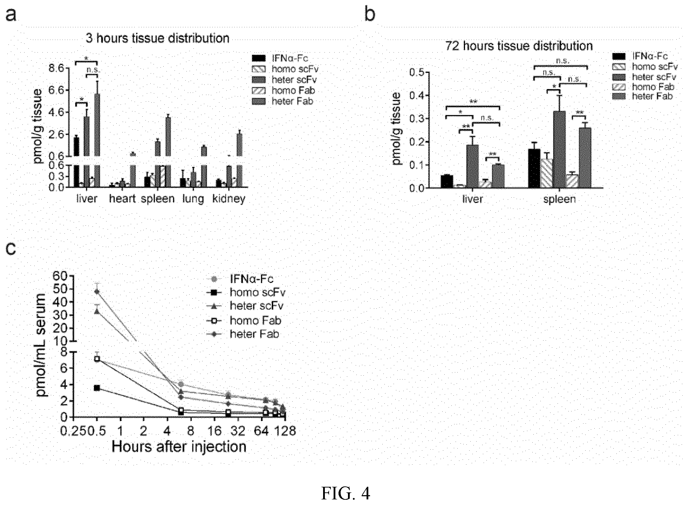

Figure 4. Heter Fab and heter scFv fusion proteins have higher accumulation and blood stability in liver. (a-c) HBV carrier mice were injected intravenously with the indicated fusion proteins (0.1687 nmol/mouse). Concentrations of each fusion protein were measured by ELISA in the tissue after 3 hours (a), the tissue after 72 hours (b) or in serum at different time points (c). Data are expressed as mean ± SEM and represent at least two independent experiments. *P < 0.05; **P < 0.01; and n.s. indicates not significant.

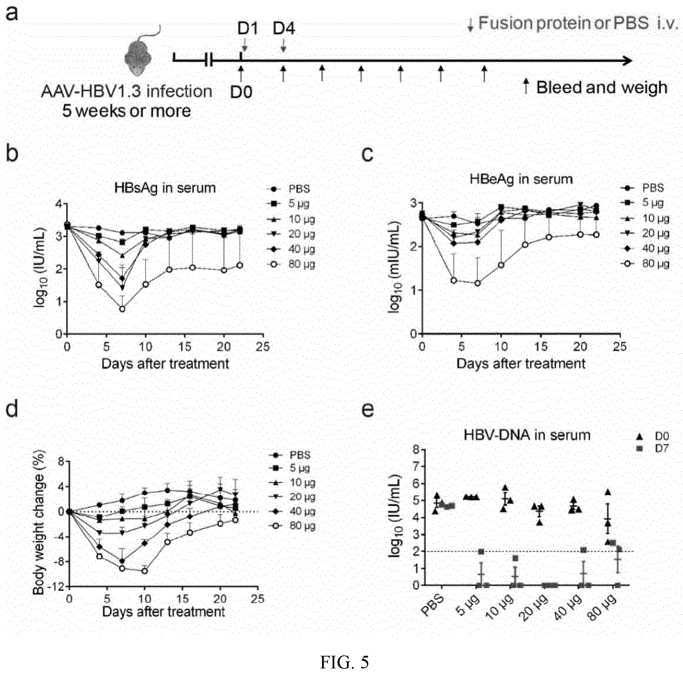

Figure 5. Heter Fab fusion protein inhibits HBV *in vivo* in a dose-dependent manner. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS or the heter Fab fusion protein at 5 µg/mouse, 10 µg/mouse, 20 µg/mouse, 40 µg/mouse, or 80 µg/mouse (6 groups in total, with 3 mice each group), and the blood was collected at the designed time points for serum collection. (b-d) The levels of HBsAg (b) and HBeAg (c) in serum at each time point were detected by ELISA, and the mice were weighed and the body weight change was calculated (d). (e) The levels of HBV-DNA in serum on days 0 and 7 were detected by real-time PCR. Data are expressed as mean± SEM and represent at least two independent experiments.

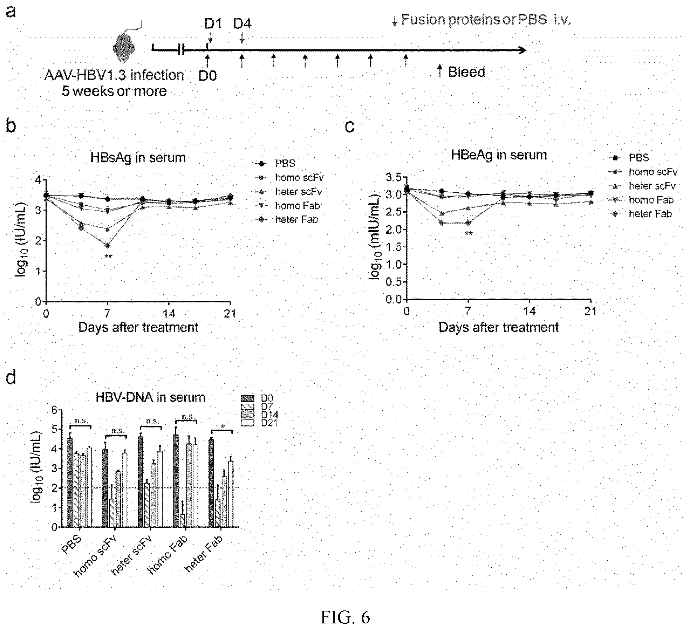

Figure 6. The *in vivo* inhibition effect of the Heter Fab or heter scFv fusion protein on HBV is better than that of homo Fab or homo scFv fusion protein on HBV. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS or the four fusion proteins (equimolar IFNα and anti-PDL1), *i.e.,* the homo scFv fusion protein (0.0844 nmol/mouse), the heter scFv fusion protein (0.1687 nmol/mouse), the homo Fab fusion protein (0.0844 nmol/mouse) or the heter Fab fusion protein (0.1687 nmol/mouse) (5 groups in total, with 3 mice each group), and the blood was collected at the designed time points for serum collection. (b, c) The levels of HBsAg (b) and HBeAg (c) in serum at each time point were detected by ELISA. (d) The levels of HBV-DNA in serum on days 0, 7, 14 and 21 were detected by real-time PCR. Data are expressed as mean ± SEM and represent at least two independent experiments. *P < 0.05; **P < 0.01; and n.s. indicates not significant.

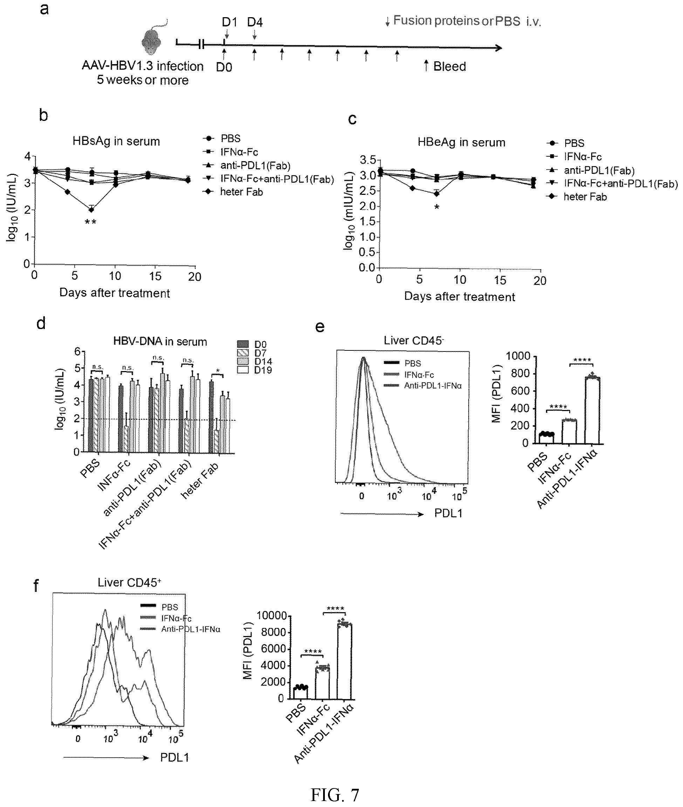

Figure 7. Heter Fab fusion protein inhibits HBV *in vivo* with the synergistic and positive feedback effects. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. (b-d) On days 1 and 4, the HBV carrier mice were injected intravenously with PBS, IFNα-Fc (0.0844 nmol/mouse), anti-PDL1(Fab) (0.0844 nmol/mouse), a mixture of IFNα-Fc and anti-PDL1(Fab) (0.0844 nmol + 0.0844 nmol/mouse) or the heter Fab fusion protein (0.1687 nmol/mouse) (5 groups in total, with 3 mice each group), and the blood was collected at the designed time points for serum collection. The levels of HBsAg (b) and HBeAg (c) in serum at each time point were detected by ELISA. (d) The levels of HBV-DNA in serum on days 0, 7, 14 and 19 were detected by real-time PCR. (e, f) On day 1, the HBV carrier mice were injected intravenously with PBS, IFNα-Fc (0.0844 nmol/mouse) or the heter Fab fusion protein (0.1687 nmol/mouse) (3 groups in total, with 6 mice each group), the liver tissues were collected after 3 days, and the levels of PDL1 on the CD45⁻ (e) and CD45⁺ (f) cells were analyzed by flow cytometry. The left panel shows a representative flow cytometry graph, and the right panel shows a statistical plot of MFI (PDL1) data. Data are expressed as mean ± SEM and represent at least two independent experiments. *P < 0.05; **P < 0.01; and n.s. indicates not significant.

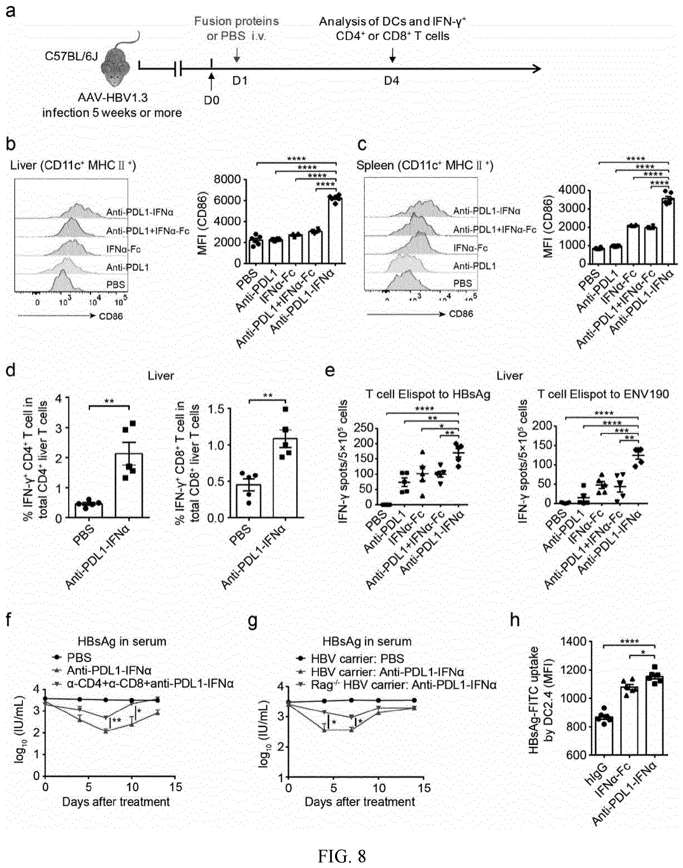

Figure 8. Heter Fab fusion protein can enhance the function of DC cells and thus promotes the function of HBsAg-specific T cells to break the immune tolerance. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On day 1, the HBV carrier mice were injected intravenously with PBS or the equimolar fusion proteins (0.1687 nmol/mouse, equimolar IFNα and anti-PDL1). After 3 days, the expressions of CD80, CD86, and MHC I on the DC cells as well as the IFN-γ⁺CD4⁺ and CD8⁺ T cells in liver and spleen were detected by flow cytometry. (b, c) Liver mononuclear cells (b) and splenocytes (c) were collected, and the expression of CD86 on the DC cells was detected by flow cytometry. (d) The liver mononuclear cells were collected, stimulated with HBsAg (ayw, 5 µg/mL) and cultured for 12 hours, and then cultured in the presence of brefeldin A (5 µg/mL) for an additional 6 hours, and the IFN-γ⁺CD4⁺ and CD8⁺ T cells were analyzed by flow cytometry. (e) The liver mononuclear cells were collected, and the HBsAg-specific T cells and the ENV190 (CD8 epitope)-specific T cells were detected by T cell ELISPOT. (f) T cells were eliminated by the intraperitoneal injection of 200 µg of anti-CD4 antibody and 200 µg of anti-CD8 antibody twice a week (injected one day before the heter Fab fusion protein treatment), and the HBV carrier mice were injected intravenously with the heter Fab fusion protein (0.1687 nmol/mouse) twice (at an interval of 3 days). T cells were not eliminated in the control groups, and the HBV carrier mice were injected intravenously with PBS or the heter Fab fusion protein (0.1687 nmol/mouse) twice (at an interval of 3 days). Blood was collected at the corresponding time points, and the level of HBsAg in serum was detected by ELISA. (g) The HBV carrier mice or Rag1^{-/-}HBV carrier mice were injected intravenously with PBS or the heter Fab fusion protein (0.1687 nmol/mouse) twice (at an interval of 3 days). Blood was collected at the corresponding time points, and the level of HBsAg in serum was detected by ELISA. (h) The DC2.4 cells were incubated with HBsAg-FITC (1 µg/mL) at 37°C for 4 hours, along with 0.391 µg/mL IFNα-Fc or 1 µg/mL heter Fab (equimolar IFNα concentration). Phagocytosis of HBsAg-FITC by DC2.4 was analyzed by flow cytometry. Data are expressed as MEAN ± SEM and represent at least two independent experiments. *P < 0.05; **P < 0.01; ***P < 0.001; and ****P < 0.0001.

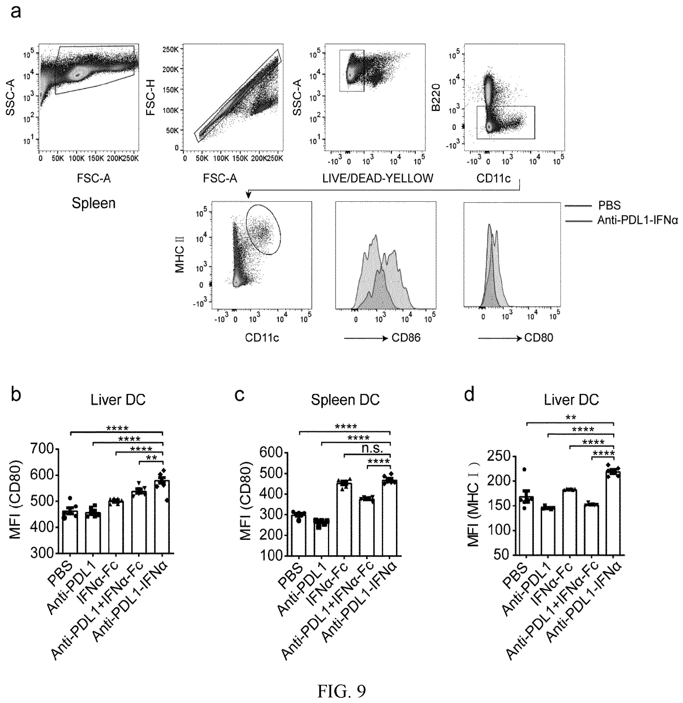

Figure 9. Heter Fab fusion protein up-regulates the expressions of CD80 and MHC I on the DC cells. (a) Gating strategy for detecting CD80 and CD86 on the DC cells using flow cytometry. (b-d) HBV carrier mice were treated as depicted in Figure 8a. On day 4, the liver mononuclear cells and splenocytes were collected, and the expressions of CD80 (b) and MHC I (d) on the DC cells in liver as well as the expression of CD80 (c) on the DC cells in spleen were analyzed by flow cytometry. Data are expressed as mean ± SEM and represent at least two independent experiments. **P < 0.01; ****P < 0.0001; and n.s. indicates not significant.

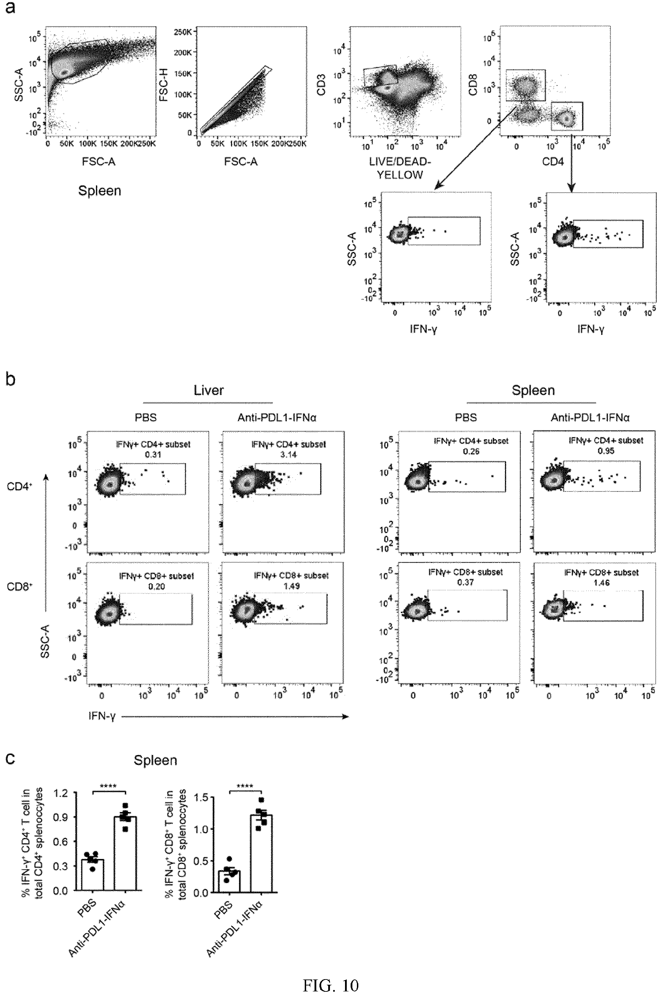

Figure 10. Heter Fab fusion protein enhances the function of HBsAg-specific T cells. (a) Gating strategy for detecting IFN-γ⁺ CD4⁺ and CD8⁺ T cells using flow cytometry. (b, c) HBV carrier mice were treated as depicted in Figure 8a. On day 4, the liver mononuclear cells and splenocytes were collected, stimulated with HBsAg (ayw, 5 µg/mL) and cultured for 12 hours, and then cultured in the presence of brefeldin A (5 µg/mL) for an additional 6 hours, and the IFN-γ⁺CD4⁺ and CD8⁺ T cells were analyzed by flow cytometry. A representative flow cytometry scatterplot (b) and a statistical plot of the proportions of the IFN-γ⁺CD4⁺ and CD8⁺ T cells in spleen (c) are shown. Data are expressed as MEAN ± SEM (data for 5 mice in each group are from two independent experiments). ****P < 0.0001.

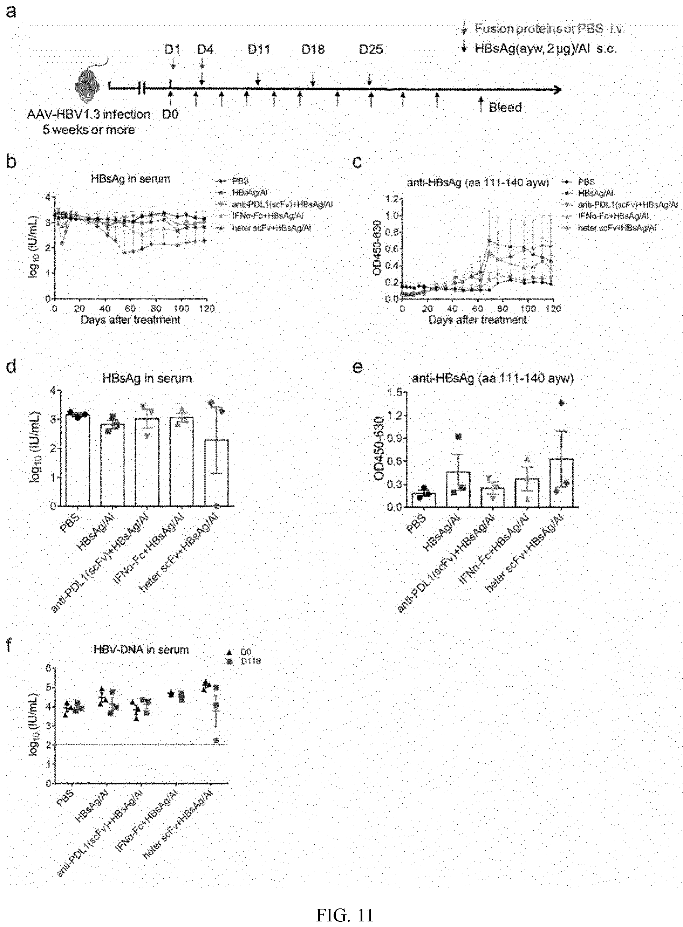

Figure 11. The Heter scFv fusion protein in combination with the HBsAg/aluminium adjuvant vaccine immunization achieves a functional cure in some HBV carrier mice. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS, IFNα-Fc (0.0844 nmol/mouse), anti-PDL1(scFv) (0.0844 nmol/mouse) or the heter scFv fusion protein (0.1687 nmol/mouse) (5 groups in total, with 3 mice each group). The fusion protein group was also given the HBsAg(ayw, 2 µg)/aluminium adjuvant vaccine for immunization on days 4, 11, 18 and 25, and a separate immunization group of the HBsAg(ayw, 2 µg)/aluminium adjuvant vaccine was set up. The blood was collected at the designed time points for serum collection. (b, c) The levels of HBsAg (b) and the anti-HBsAg antibodies (c) in serum at each time point were detected by ELISA. (d, e) The levels of HBsAg (d) and the anti-HBsAg antibodies (e) in serum at the end of the experiment (day 118) were detected by ELISA. (f) The levels of HBV-DNA in serum on day 0 and at the end of the experiment (day 118) were detected by real-time PCR. In panels (d-f), one mouse in the heter scFv+HBsAg/aluminium adjuvant group achieved a functional cure (*i.e.,* the serum HBsAg and HBV-DNA clearance accompanied by the production of anti-HBsAg). Data are expressed as mean ± SEM and represent at least two independent experiments.

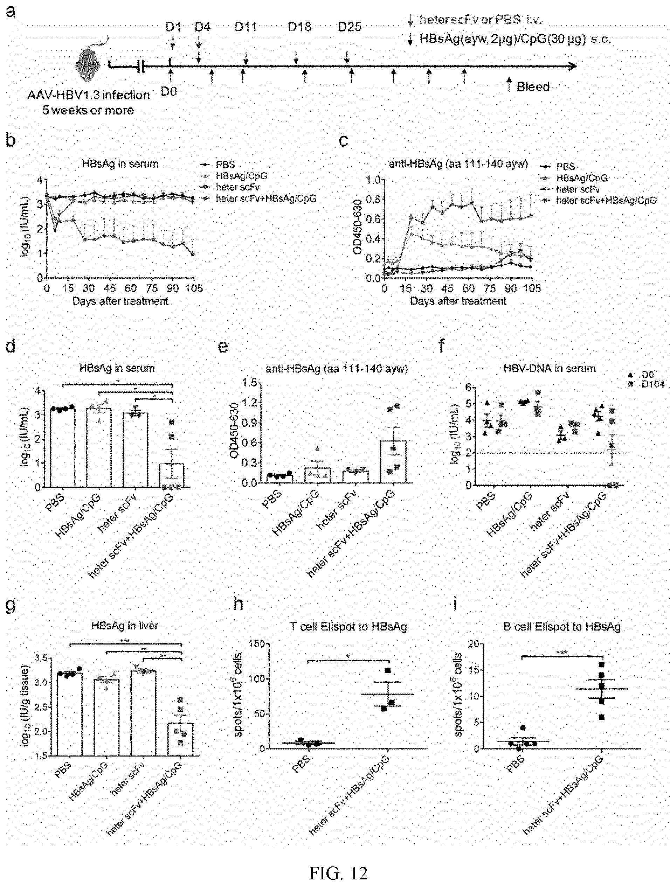

Figure 12. The Heter scFv fusion protein in combination with the HBsAg/CpG vaccine achieves a functional cure in most HBV carrier mice. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS or the heter scFv fusion protein (0.1687 nmol/mouse) (4 groups in total). Two of the groups (the heter scFv+HBsAg/CpG and HBsAg/CpG groups) were immunized with HBsAg (ayw, 2 µg) and CpG adjuvant for a total of 4 times on days 4, 11, 18 and 25, and the blood was collected at the designed time points for serum collection. (b, c) The levels of HBsAg (b) and the anti-HBsAg antibodiess (c) in serum at each time point were detected by ELISA. (d, e) The levels of HBsAg (d) and the anti-HBsAg antibodies (e) in serum at the end of the experiment (day 104) were detected by ELISA. (f) The levels of HBV-DNA in serum on day 0 and at the end of the experiment (day 104) were detected by real-time PCR. (g) The level of HBsAg in liver at the end of the experiment (day 104) was detected by ELISA. In panels (d-f), three mice in the heter scFv+HBsAg/CpG group achieved a functional cure (*i.e.,* the serum HBsAg and HBV-DNA clearance accompanied by the production of anti-HBsAg antibodies). (h, i) At the end of the experiment (day 104), the splenic lymphocytes were collected from mice in the PBS group and the combined treatment group (heter scFv+HBsAg/CpG) for the detection of HBsAg-specific T and B lymphocytes. 1 × 10⁶ cells were plated per well, and stimulated with 5 µg/mL HBsAg (ayw) for 48 hours. The specific T cell responses were detected by T cell ELISPOT (h), and the specific B cell responses were detected by B cell ELISPOT (i). Data are expressed as mean ± SEM and represent at least two independent experiments. *P < 0.05; **P < 0.01; and ***P < 0.001.

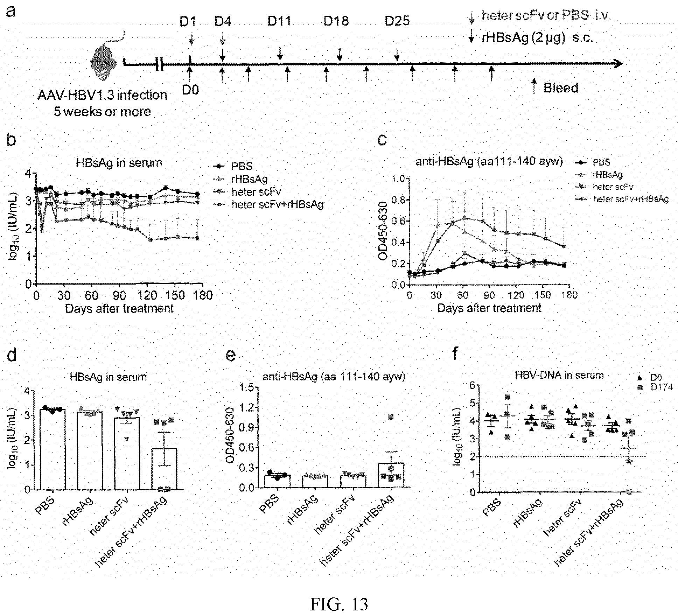

Figure 13. The Heter scFv fusion protein in combination with a commercial prophylactic hepatitis B vaccine achieves a functional cure in some HBV carrier mice. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS or the heter scFv fusion protein (0.1687 nmol/mouse) (4 groups in total). Two of the groups (the heter scFv+HBsAg/CpG and HBsAg/CpG groups) were immunized with the commercial prophylactic hepatitis B vaccine rHBsAg (adw, 2 µg) for a total of 4 times on days 4, 11, 18 and 25, and the blood was collected at designed time points for serum collection. (b, c) The levels of HBsAg (b) and anti-HBsAg antibodies (c) in serum at each time point were detected by ELISA. (d, e) The levels of HBsAg (d) and anti-HBsAg antibodies (e) in serum at the end of the experiment (day 174) were detected by ELISA. (f) The levels of HBV-DNA in serum on day 0 and at the end of the experiment (day 174) were detected by real-time PCR. In panels (d-f), two mice in the heter scFv+rHBsAg group achieved a functional cure (*i.e.,* the serum HBsAg and HBV-DNA clearance accompanied by the production of anti-HBsAg antibodies). Data are expressed as mean ± SEM and represent at least two independent experiments.

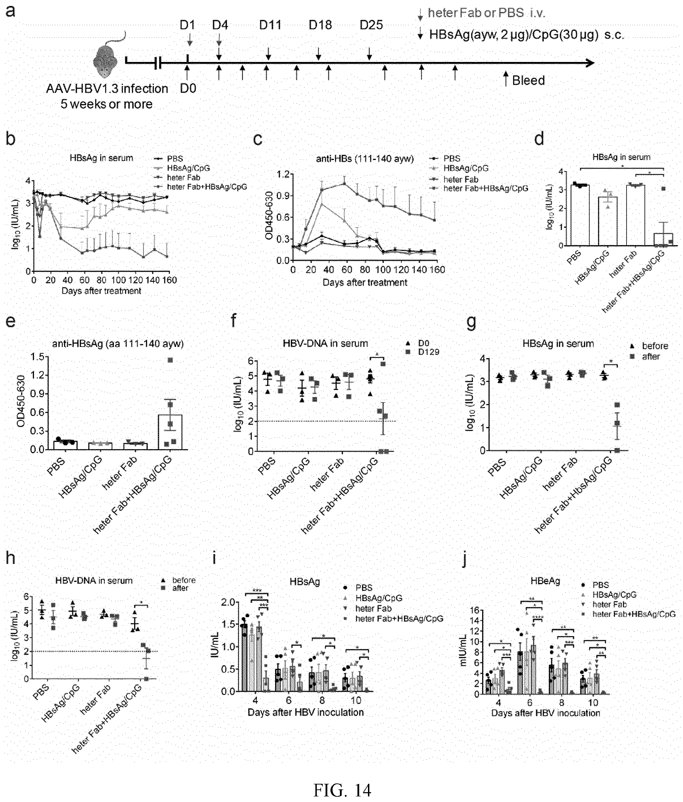

Figure 14. The Heter Fab fusion protein in combination with a HBsAg/CpG vaccine achieves a functional cure in most HBV carrier mice. (a) C57BL/6J male mice (4-6 weeks old) were injected intravenously with 1 × 10¹¹ GC AAV-HBV1.3/mouse, and those with the stable serum HBsAg levels higher than 1000 IU/mL after 5 weeks were used as the HBV carrier mice for the subsequent experiments and the day was recorded as day 0. On days 1 and 4, the HBV carrier mice were injected intravenously with PBS or the heter Fab fusion protein (0.1687 nmol/mouse) (4 groups in total). Two of the groups (the heter Fab+HBsAg/CpG and HBsAg/CpG groups) were immunized with HBsAg (ayw, 2 µg) and CpG adjuvant for a total of 4 times on days 4, 11, 18 and 25, and the blood was collected at the designed time points for serum collection. (b, c) The levels of HBsAg (b) and anti-HBsAg antibodies (c) in serum at each time point were detected by ELISA. (d, e) The levels of HBsAg (d) and the anti-HBsAg antibodies (e) in serum on day 157 were detected by ELISA. (f) The levels of HBV-DNA in serum on days 0 and 157 were detected by real-time PCR. In panels (d-f), three mice in the heter Fab+HBsAg/CpG group achieved a functional cure (*i.e.,* the serum HBsAg and HBV-DNA clearance accompanied by the production of anti-HBsAg antibodies). (g, h) HBV carrier mice without any treatment were injected intravenously with the serum from 3 functionally cured mice in the combined treatment group (*i.e.,* the heter Fab+HBsAg/CpG group) and the seum from mice in the other groups, with 100 µL per mouse. The levels of HBsAg (g) and HBV-DNA (h) in serum of these tested HBV carrier mice before and 24 hours after the injection were detected by ELISA and real-time PCR, respectively. (i, j) HepG2-NTCP cells were incubated with 1 × 10⁷ vg HBV in serum of the HBV carrier mice treated with PBS, HBsAg/CpG, heter Fab, or heter Fab+HBsAg/CpG (n = 5/group). After 24 hours of incubation, the HepG2-NTCP cells were washed three times with the culture medium and then cultured in a maintenance medium, and the supernatant was collected and the medium was replaced with the fresh maintenance medium every 2 days. The levels of HBsAg (i) and HBeAg (j) in the supernatant were detected by ELISA. Data are expressed as mean ± SEM and represent at least two independent experiments. *P < 0.05; **P < 0.01; ***P < 0.001; and ****P < 0.0001.

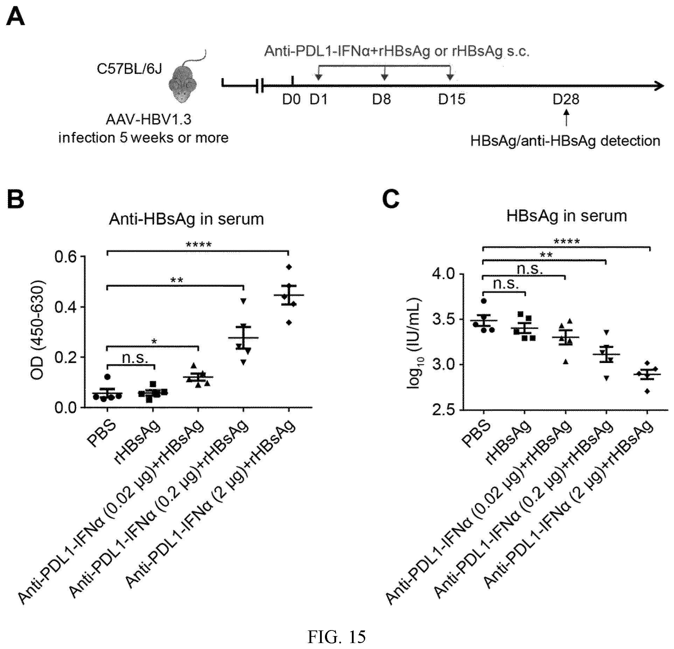

Figure 15. HBV chronically infected mice are subcutaneously immunized with the mixture of Heter Fab fusion protein and commercial prophylactic hepatitis B vaccine, wherein the mixture acts as a therapeutic vaccine. (A) HBV carrier mice were subcutaneously immunized with the heter Fab fusion protein (3 concentration gradients of 0.02 µg, 0.2 µg and 2 µg) mixed with the commercially available prophylactic hepatitis B vaccine (2 µg) for 3 times at 1-week interval. Serum of mice was collected for detection of the levels of anti-HBsAg antibodies and HBsAg. (B) The levels of anti-HBsAg antibodies in serum of mice. (C) The level of HBsAg antigen in serum of mice. Data are expressed as mean ± SEM and represent at least two independent experiments. *P < 0.05; and **P < 0.01.

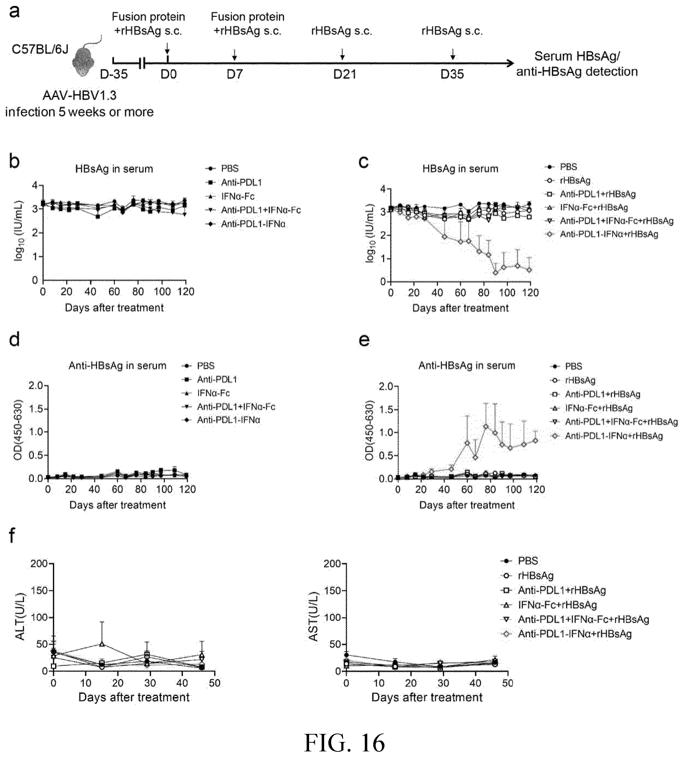

Figure 16. Anti-PDL1-IFNα acts as a targeted adjuvant to induce the HBV carrier mice to overcome the immune tolerance to rHBsAg vaccine. (a) Dosing regimens of equimolar amounts (0.01687 nmol, calculated on the basis of a single subunit) of the fusion proteins administered subcutaneously alone or mixed with 1 µg of rHBsAg vaccine for a subcutaneous immunotherapy of the HBV carrier mice. (b, d) The levels of HBsAg (b) and anti-HBsAg antibodies (d) in serum after the subcutaneous administration of fusion protein alone were measured by ELISA. (c, e) The levels of HBsAg (c) and anti-HBsAg antibodies (e) in serum after the subcutaneous administration of fusion protein mixed with the rHBsAg vaccine were detected by ELISA. (f) The levels of ALT and AST in serum were detected by the kits. Data are expressed as mean ± SEM.

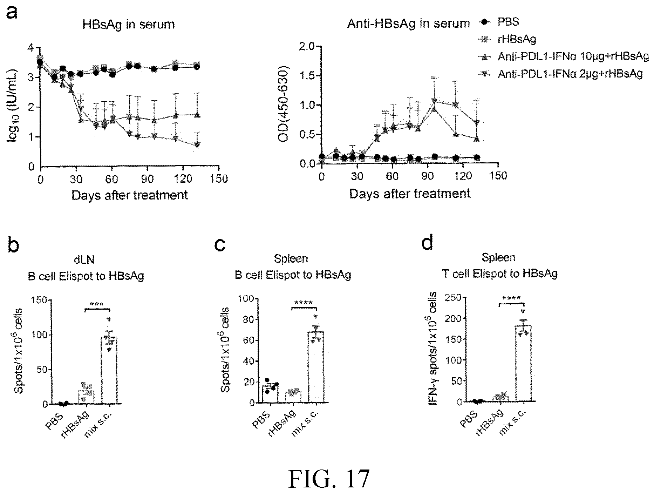

Figure 17. Anti-PDL1-IFNα, as a targeted adjuvant, can effectively promote the immunity of rHBsAg vaccine and achieve the HBsAg seroconversion. (a) The treatment regimen of HBV carrier mice was the same as that shown in Figure 16(a), and the levels of HBsAg and anti-HBsAg antibodies in serum at each time point were detected by ELISA. (b-d) At the end of the experiment, the draining lymph nodes and spleens were collected and prepared into the single-cell suspensions. HBsAg-specific B cells in the draining lymph nodes (b) and spleens (c) were detected by B-cell ELISPOT, and HBsAg-specific T cells in spleens (d) were detected by T-cell ELISPOT. Data are expressed as mean ± SEM (a) or mean ± SEM (b-d).

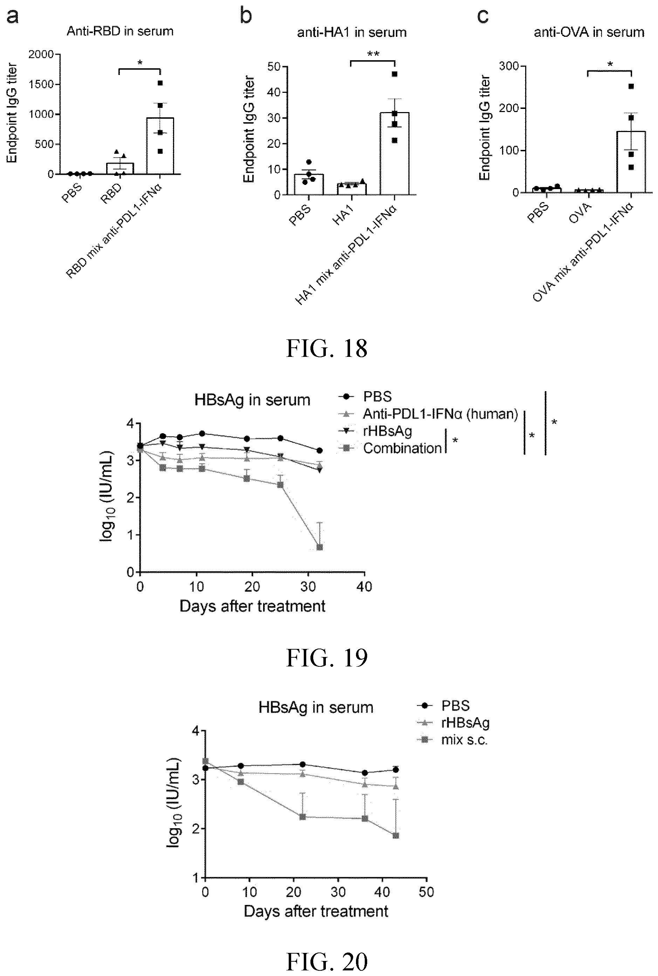

Figure 18. Anti-PDL1-IFNα, as an immune adjuvant, can effectively enhance the immunity against a receptor-binding region (RBD) antigen of SARS-CoV-2, an HA1 antigen of influenza virus and an OVA antigen. Wild-type mice were immunized subcutaneously with the mixture of anti-PDL1-IFNα (2 µg/mouse) and the RBD antigen (3.4 µg/mouse) or the RBD antigen alone (3.4 µg/mouse). Ten days after the initial immunization with the anti-PDL1-IFN fusion protein, the serum from the immunized mice was collected, and the levels of anti-RBD antibodies in serum were detected by ELISA (Figure 18a). Wild-type mice were immunized subcutaneously with the mixture of anti-PDL1-IFNα (2 µg/mouse) and HA1 antigen (2 µg/mouse) or the HA1 antigen alone (2 µg/mouse). Ten days after the initial immunization, the serum from the immunized mice was collected and the levels of anti-HA1 antibodies in serum were detected by ELISA (Figure 18b). Wild-type mice were subcutaneously immunized with the mixture of anti-PDL1-IFNα (2 µg/mouse) and OVA antigen (2 µg/mouse) or the OVA antigen alone (2 µg/mouse). Ten days after the initial immunization, the serum from the immunized mice was collected and the levels of anti-OVA antibodies in serum were detected by ELISA (Figure 18c). Data are expressed as mean ± SEM.
Figure 19. The anti-PDL1-IFNα (human) fusion protein in combination with the rHBsAg vaccine achieves a decrease in the serum HBsAg level of the HBV carrier mice. The mouse IFNα4 in the anti-PDL1-IFNα fusion protein was replaced with human-derived IFNα2 (Q124R) which can bind to mouse IFNAR, and this fusion protein containing human interferon was named as anti-PDL1-IFNα (human). On days 1 and 4, the HBV carrier mice were injected intravenously with PBS or the anti-PDL1-IFNα (human) fusion protein (20 µg/mouse), respectively (2 groups each, 4 groups in total). Of these, 1 group from the mice injected with PBS and 1 group from the mice injected with the anti-PDL1-IFNα (human) fusion protein (2 groups in total) were selected and subcutaneously immunized with the rHBsAg vaccine (1 µg/mouse) (*i.e.,* the "anti-PDL1-IFNα (human) + rHBsAg" and the "rHBsAg vaccine alone + PBS" groups) and aluminium adjuvant for a total of 4 times on days 4, 11, 18, and 25, and the blood was collected at the designed time points for serum collection. The levels of HBsAg in serum at each time point were detected by ELISA (Figure 19). Data are expressed as mean ± SEM.
Figure 20. Anti-PDL1-IFNα (human), administered subcutaneously as a targeted immune adjuvant, can effectively promote the immunity of rHBsAg vaccine and achieves a decrease in the serum HBsAg level. HBV carrier mice were subcutaneously immunized with a mixture of anti-PDL1-IFNα (human) (2 µg/mouse) and rHBsAg (1 µg/mouse) with the same specific treatment regimen as that shown in Figure 16(a), and the levels of HBsAg in serum at each time point were detected by ELISA (Figure 20). Data are expressed as mean ± SEM.

### EXAMPLES

The invention is described in further details by the following Examples, but it should be understood that the invention is not limited by the following Examples.

### Experimental materials and methods

### Mouse and construction of the HBV carrier mouse model

Male (4-6 weeks old) C57BL/6J mice were purchased from SiPeiFu (Beijing) Biotechnology Co., Ltd. (Beijing, China). All experimental mice were housed in an ABSL-2 (Biosafety Level II) infected animal facility at the SPF (Specific Pathogen Free) grade. All experiments were conducted in compliance with the provisions of the animal experiment control number ABSL-2-201902 of the Institute of Biophysics, Chinese Academy of Sciences. Animal care and experiments were performed according to the guidelines of the Institute of Biophysics, Chinese Academy of Sciences, following the protocols approved by IACUC (Institutional Laboratory Animal Care and Use Committee).

The AAV-HBV1.3 virus used to construct the HBV carrier experimental animal model was purchased from Guangzhou PackGene Biotechnology Co., Ltd. (Guangzhou, China), with the serotype of ayw subtype and the genotype of type D. Mice were infected by the tail vein injection at a dose of 1×10¹¹ GC/mouse (diluted in 200 µL of saline). The stable infected mice (with the level of serum HBsAg ≥1000 IU/mL, *i.e.,* the HBV carrier mice) were used for the experiments after 5 weeks. The levels of HBsAg, HBeAg, the anti-HBsAg antibodies and HBV-DNA in serum were measured by the ocular vein blood collection in the experiments.

### Cell lines and main reagents

The L929 cell line was a mouse fibroblast (purchased from ATCC, USA), which was used for the detection of the IFN bioactivity *in vitro.* HepG2-NTCP (purchased from Qingqi (Shanghai) Biotechnology Development Co., Ltd.) was used for the HBV infection and inhibition assay *in vitro.* The levels of HBsAg and HBeAg in mouse serum were determined using an ELISA kit (purchased from Shanghai Kehua Bioengineering Co., Ltd.; Shanghai, China). A polypeptide with the amino acids at positions 111-140 of the HBsAg protein (ayw serotype, the amino acid sequence was: PGSSTTSTGPCRTCMTTAQGTSMYPSCCCT) was synthesized by Shanghai GL Biochemical Co., Ltd. (Shanghai, China), and this polypeptide was used to coat a 96-well plate to determine the level of the anti-HBsAg antibodies in mouse serum. The level of HBV-DNA in mouse serum was quantified using a kit purchased from Sansure Biotechnology Co., Ltd. (Changsha, China) by real-time PCR. Liver DNA of mouse was extracted using a gDNA kit (Tiangen Biotech, China). The levels of HBV-DNA, 3.5kb RNA, and total RNA in liver of mouse were determined using a SYBR Premix Ex Taq kit purchased from TaKaRa (Japan) by real-time PCR. Prophylactic hepatitis B vaccine was purchased from Dalian Hissen Biopharmaceutical Co., Ltd. (Dalian, China).The HBsAg (ayw) protein (purchased from Meridian Life Science, USA) was used for the vaccine immunization and the enzyme-linked immunospot (ELISPOT) experiments in mice, and the ELISPOT kit was purchased from BD company (USA). Aluminium adjuvant (Alu-Gel-S) was purchased from Serva Electrophoresis GmbH (Germany), and the CpG-1826 adjuvant was synthesized by Shanghai Generay Biotechnology Co., Ltd. (Shanghai, China). Human IgG Fc was quantitatively detected by ELISA using the goat anti-human IgG and goat anti-HRP antibodies (Beijing CoWin Biotechnology Co., Ltd; Beijing, China).

### Construction and activity analysis of anti-PDL1-IFN and the control fusion proteins

### a. Construction of the fusion proteins

Heter Fab heterodimer: The cDNA sequence encoding murine IFNα4 was cloned and inserted into the N-terminus of the nucleotide sequence encoding the human IgG1 Fc fragment through a (G4S)₄ linker to obtain the nucleotide sequence encoding mIFNα4-Fc (SEQ ID NO.2), and then the nucleotide sequence encoding mIFNα4-Fc (SEQ ID NO.2) was cloned into a pEE6.4 vector (Lonza) and expressed to obtain the first polypeptide (SEQ ID NO. 1) of the heterodimer mIFNα4-Fc. The nucleotide sequence encoding the Fab fragment of the heavy chain of the anti-PDL1 antibody was cloned and inserted into the N-terminus of the human IgG1 Fc fragment to obtain the nucleotide sequence encoding the anti-PDL1 heavy chain Fab-Fc (SEQ ID NO.6). The nucleotide sequence encoding the anti-PDL1 heavy chain Fab-Fc (SEQ ID NO.6) was cloned into a pEE12.4 vector (Lonza) and expressed to obtain the second polypeptide (i.e., the anti-PDL1 heavy chain Fab-Fc) (SEQ ID NO.5) of the heterodimer. The nucleotide sequence encoding the light chain of the anti-PDL1 antibody (SEQ ID NO.4) was cloned into a pEE12.4 vector (Lonza) and expressed to obtain the third polypeptide (i.e., the light chain of the anti-PDL1 antibody) (SEQ ID NO.3) of the heterodimer. Heterodimerization of IFNα and the PDL1-binding polypeptide was carried out using the previously reported knobs-to-holes technology. Plasmids were transiently transfected into 293F cells at a ratio of 2:1:2. Supernatants were collected on day 7 after transfection. The fusion protein was purified using a protein A-agarose gel column according to the operation manual (Repligen company).

Heter scFv heterodimer: The cDNA sequence encoding murine IFNα4 was cloned and inserted into the N-terminus of the nucleotide sequence encoding the human IgG1 Fc fragment through a (G4S)₄ linker to obtain the nucleotide sequence encoding mIFNα4-Fc (SEQ ID NO.2), and then the nucleotide sequence encoding mIFNα4-Fc (SEQ ID NO.2) was cloned into a pEE6.4 vector (Lonza) and expressed to obtain the first polypeptide mIFNα4-Fc (SEQ ID NO. 1) of the heterodimer. The nucleotide sequences encoding the variable regions of the light and heavy chains of the PDL1-binding polypeptide (scFv) (YW243.55.S70) were synthesized according to the patent (Patent No: US8217149B2). The nucleotide sequences encoding the variable regions of the light and heavy chains were linked through a GGGGSGGGGSGGGGS linker, and the nucleotide sequence encoding a human IgG1 Fc fragment was inserted into the C-terminus of the nucleotide sequence encoding the heavy chain to obtain a nucleotide sequence encoding scFv(PDL1)-Fc (SEQ ID NO.10). Then, the nucleotide sequence encoding scFv(PDL1)-Fc (SEQ ID NO.10) was cloned into a pEE12.4 vector (Lonza) and expressed to obtain the second polypeptide scFv(PDL1)-Fc (SEQ ID NO.9) of the heterodimer. Heterodimerization of IFNα and the PDL1-binding polypeptide was carried out using the previously reported knobs-to-holes technology. Plasmids were transiently transfected into 293F cells at a ratio of 2:1. Supernatants were collected on day 7 after transfection. The fusion protein was purified using a protein A-agarose gel column according to the operation manual (Repligen company).

Homo Fab homodimer: The cDNA sequence encoding murine IFNα4 and the nucleotide sequence encoding the Fab fragment of the heavy chain of the anti-PDL1 antibody were connected by connecting the C-terminus of the cDNA sequence encoding murine IFNα4 to the N-terminus of the nucleotide sequence encoding the Fab fragment of the heavy chain of the anti-PDL1 antibody, and then inserted into the N-terminus of the nucleotide sequence encoding the human IgG1 Fc fragment (WT) to obtain a nucleotide sequence encoding the homo Fab homodimer (SEQ ID NO.8). The nucleotide sequence encoding the homo Fab homodimer (SEQ ID NO.8) was cloned into a pEE12.4 vector (Lonza) and expressed to obtain the first and second polypeptides (SEQ ID NO.7) of the homodimeric fusion protein. The nucleotide sequence encoding the light chain of the anti-PDL1 antibody (SEQ ID NO.4) was cloned into a pEE12.4 vector (Lonza) and expressed to obtain the third and fourth polypeptides (SEQ ID NO.3) of the homodimeric fusion protein. Homodimeric protein was formed spontaneously by the dimerization of Fc (WT) after transfection of the plasmids at a ratio of 1:1. Supernatants were collected on day 7 after transfection. The fusion protein was purified using a protein A-agarose gel column according to the operation manual (Repligen company).

Homo scFv homodimer: The cDNA sequence encoding murine IFNα4 and the nucleotide sequence encoding scFv(PDL1)-Fc(WT) were connected by connecting the C-terminus of the cDNA sequence encoding murine IFNα4 to the N-terminus of the nucleotide sequence encoding scFv(PDL1)-Fc(WT), and a nucleotide sequence encoding the homo scFv homodimer (SEQ ID NO.12) was obtained. The nucleotide sequence encoding the homo scFv homodimer (SEQ ID NO.12) was cloned into a pEE12.4 vector (Lonza) and expressed to obtain the first and second polypeptides (SEQ ID NO.11) of the homodimeric fusion protein. Homodimeric protein was formed spontaneously by the dimerization of Fc (WT) after transfection. Supernatants were collected on day 7 after transfection. The fusion protein was purified using the protein A-agarose gel column according to the operation manual (Repligen company).

In the present invention, it is also possible to replace the Fc region in the dimer described above with an Fc mutant such as a Fc region in the no ADCC form, or it is also possible to replace mouse IFNα4 with human IFNα2. A person skilled in the art can expect that none of the described changes will substantially affect the effect of the present invention. The present inventors exemplarily replaced the Fc region of the Heter Fab heterodimer with the Fc region in the no ADCC form, and/or replaced mouse IFNα4 with human IFNα2, and named the resulting fusion proteins as Anti-PDL1-IFNα (no ADCC) and anti-PDL1-IFNα (human), respectively.

IFNα-Fc: The cDNA sequence encoding murine IFNα4 was cloned and inserted into the N-terminus of the nucleotide sequence encoding the human IgG1 Fc fragment (WT) through a (G4S)₄ linker to obtain a nucleotide sequence encoding IFNα-Fc (SEQ ID NO.14). The nucleotide sequence encoding IFNα-Fc (SEQ ID NO.14) was cloned into a pEE6.4 vector (Lonza) and expressed to obtain the first and second polypeptides IFNα-Fc (SEQ ID NO.13). Homodimeric protein was formed spontaneously by the dimerization of Fc (WT) after transfection. Supernatants were collected on day 7 after transfection. The fusion protein was purified using the protein A-agarose gel column according to the operation manual (Repligen company).

Complete anti-PDL1(Fab) antibody: The nucleotide sequence encoding the Fab fragment of the heavy chain of the anti-PDL1 antibody was cloned and inserted into the N-terminus of the nucleotide sequence encoding the human IgG1 Fc fragment (WT) to obtain a nucleotide sequence encoding the anti-PDL1 heavy chain Fab-Fc (SEQ ID NO.16). The nucleotide sequence encoding the anti-PDL1 heavy chain Fab-Fc (SEQ ID NO.16) was cloned into a pEE12.4 vector (Lonza) and expressed to obtain the anti-PDL1 heavy chain Fab-Fc polypeptide (SEQ ID NO.15). The nucleotide sequence encoding the light chain of the anti-PDL1 antibody (SEQ ID NO.4) was cloned into a pEE12.4 vector (Lonza) and expressed to obtain the light chain polypeptide of the anti-PDL1 antibody (SEQ ID NO.3). Dimeric protein was formed spontaneously by the dimerization of Fc (WT) after transfection of the plasmids at a ratio of 2:3. Supernatants were collected on day 7 after transfection. The fusion protein was purified using a protein A-agarose gel column according to the operation manual (Repligen company).

Anti-PDL1(scFv) antibody: The nucleotide sequences encoding the variable regions of the light and heavy chains of the PDL1-binding polypeptide (scFv) (YW243.55.S70) were synthesized according to the patent (Patent No: US8217149B2). The nucleotide sequences encoding the variable regions of the light and heavy chains were linked through a GGGGSGGGGSGGGGS linker, and a nucleotide sequence encoding the human IgG1 Fc fragment (WT) was inserted into the C-terminus of the nucleotide sequence encoding the heavy chain to obtain a nucleotide sequence encoding anti-(PDL1) antibody (scFv) (SEQ ID NO.18). Then, the nucleotide sequence encoding anti-(PDL1) antibody (scFv) (SEQ ID NO. 18) was cloned into a pEE12.4 vector (Lonza) and expressed to obtain the first and second polypeptides anti-(PDL1) (scFv) (SEQ ID NO.17). Dimeric protein was formed spontaneously by the dimerization of Fc (WT) after transfection. Supernatants were collected on day 7 after transfection. The fusion protein was purified using a protein A-agarose gel column according to the operation manual (Repligen company).

**b*. In vitro* functional experiments:** The purity of the fusion proteins was detected by SDS-PAGE. Vesicular Stomatitis Virus-Green Fluorescent Protein (VSV-GFP) (wherein said VSV-GFP is a GFP-expressing murine vesicular stomatitis virus, wherein said murine vesicular stomatitis virus is a virus conventionally used in the field (purchased from VectorBulider Biotechnology Co., Ltd.) was used to infect the fusion protein-incubated L929 cells (a murine fibroblast) (purchased from ATCC, USA) to test the anti-viral biological activity of IFN. Biolayer interferometry (BLI) was used to analyze the affinities of IFN/IFN receptor, and anti-PDL1 antibody/PDL1. Flow cytometry (FACS) was used to analyze the binding ability of anti-PDL1-IFN to IFNAR⁺ and PDL1⁺ cells.

L929, which is sensitive to the VSV-GFP infection, was used to determine the bioactivity of IFN. Cells were incubated with the serial dilutions of IFNα-Fc or four anti-PDL1-IFN fusion proteins overnight at 37°C. The next day, cells were infected with VSV-GFP with MOI = 5 and incubated for an additional 16 hours. Cells were then collected and fixed with 4% PFA. Data were acquired using the FACS LSRFortessa flow cytometer (BD Biosciences) and analyzed using the FlowJo software (TreeStar). GFP⁺ cells are defined as the virus-infected cells, and MFI is use to characterize the intensity of viral replication.

The binding affinity of the anti-PDL1-IFN fusion protein to the PDL1 protein was measured on a Octet Red96 system (Pall ForteBio) using biolayer interferometry (BLI). Binding experiments were performed in binding buffer (PBS, 0.05% Tween 20, pH 7.4) at 37°C. The SA sensor in a 96-well plate was first loaded with the biotinylated PDL1 protein (at a concentration of 10 µg/mL), then the SA sensor was moved to the binding wells to generate a baseline, further moved to the sample protein wells (at the concentrations of 200 nM, 100 nM, 50 nM, 25 nM, 12.5 nM, and 6.25 nM) for 120 s, and finally immersed into the wells with the binding buffer for dissociation for 180 seconds. Affinity constants were calculated by a dynamic analytical model, K_{D} = k_{d}/kₐ (d, dissociation; a, association).

**c. *In vivo* pharmacokinetic analysis:** determination of *in vivo* tissue distribution and blood concentration of the anti-PDL1-IFN fusion proteins.

Determination of tissue distribution: HBV carrier mice were injected intravenously with IFNα-Fc or four anti-PDL1-IFN fusion proteins (0.1687 nmol/mouse), and then the tissues were collected by perfusion after 3 or 72 hours, and homogenized and centrifuged to collect the supernatants. The concentration of the fusion protein in the supernatant of the homogenate of each tissue was determined by ELISA.

Determination of blood concentration: HBV carrier mice were injected intravenously with IFNα-Fc or four anti-PDL1-IFN fusion proteins (0.1687 nmol/mouse). The blood was collected at 0.5, 6, 24, 72, 96, and 120 hours, and centrifuged to collect the serum. The concentration of the fusion protein in serum at each time point was determined by ELISA.

### d. In vivo toxicity analysis

The drug was administered within the gradient measurement range, the body weights of the experimental mice was recorded, and the body weight changes of the mice was calculated.

### Effect of the anti-PDL1-IFN fusion protein on DC cells

A mouse model of chronic HBV infection (the HBV carrier mouse) was constructed, and anti-PDL1-IFN was administered intravenously. The levels of the surface co-stimulatory molecules (CD80 and CD86) as well as MHC I molecule on the DC cells in mouse spleen and liver were detected by flow cytometry (FACS), and the phagocytosis of HBsAg antigen by the DC cells was detected.

### Effect of the anti-PDL1-IFN on the adaptive immune cells

The levels of IFN-γ secreted by T cells in the spleen and liver of the HBV carrier mice after the administration of anti-PDL1-IFN were detected by the method of cytokine intracellular staining.

### Analysis of the virological indices of the HBV carrier mice after treatment

a. The levels of HBsAg, the anti-HBsAg antibodies, HBeAg and HBV-DNA in serum were detected;
b. The level of HBsAg in liver was detected.

### Combined therapy

Anti-PDL1-IFN fusion protein in combination with the HBsAg vaccine were used to treat the HBV carrier mice. The protocol of the combined therapy was explored according to the following 2 treatment procedures, and the levels of the HBsAg antigen, the anti-HBsAg antibodies and HBV-DNA in serum were analyzed.
a. Anti-PDL1-IFN fusion protein was administered intravenously followed by a subcutaneous immunization with the commercial HBsAg vaccine;
b. Subcutaneous immunization was carried out with the mixture of anti-PDL1-IFN fusion protein and the commercial HBsAg vaccine.

### In vivo neutralization assay and in vitro blocking assay against HBV in serum

*In vivo* neutralization assay against HBV: The serum from the PBS, HBsAg/CpG, heter Fab, heter Fab+HBsAg/CpG treated HBV carrier mice was collected and injected intravenously into the untreated HBV carrier mice, with 100 µL of serum per mouse. The serum of these mice was collected 24 hours later, and the levels of HBsAg and HBV-DNA in the serum before and after treatment were detected by ELISA and real-time PCR, respectively.

*In vitro* assay of blocking the HBV infection: 1×10⁷ vg HBV was inoculated into the HepG2-NTCP cells in a 48-well plate (8×10⁴ cells per well), with the addition of serum from the HBV carrier mice treated with PBS, HBsAg/CpG, heter Fab, and heter Fab+HBsAg/CpG, and cultured for 24 hours. The cells were washed 3 times with the culture medium and then cultured in a maintenance medium. The maintenance medium was William's E medium (Gibco) supplemented with 5 µg/mL transferrin, 5 ng/mL sodium selenite, 3 µg/mL insulin (insulin-transferrin-sodium selenite; Corning), 2 mM L-glutamine, 10 ng/mL epidermal growth factor (Sigma- Aldrich), 2% dimethyl sulfoxide, 100 U/mL penicillin, and 100 µg/mL streptomycin. The supernatants were collected and replaced with the fresh maintenance medium every 2 days. The levels of HBsAg and HBeAg in the supernatants collected at different time points were detected by ELISA.

### ELISPOT detection of HBsAg antigen-specific T and B cells

Single-cell suspensions were prepared by collecting the mouse lymph nodes, spleens and livers at the end of the animal experiments. T-cell ELISPOT was performed to determine the production of IFN-γ using the IFN-γ ELISPOT kit according to the manufacturer's operation protocol (BD Biosciences). The operation protocol of B-cell ELISPOT: The culture plates were coated with HBsAg diluted with PBS (ayw-type, at the final concentration of 5 µg/mL) with 100 µL per well overnight at 4°C. The liquid was discarded. Each well was washed once with 200 µL of culture medium, and then blocked by adding 200 µL of culture medium at 37°C for 2 hours. The single cell suspension was plated into a 96-well culture plate, and then incubated in an incubator at 37°C for 5-6 hours. Each well was washed for 5 times with the washing buffer, added with 100 µL of a detection antibody (biotin-anti mouse IgG) and incubated at room temperature for 2 hours. Each well was washed for 5 times with the washing buffer, added with 100 µL of HRP-labelled streptavidin and incubated at room temperature for 1 hour. Each well was washed for 5 times with the washing buffer, and added with 100 µL of substrate, subjected to color development for 5-20 minutes. The plate was rinsed with water to terminate the color development. The spots formed were counted using a fluorescence enzyme-linked spot analyzer (Cellular Technology Ltd., USA).

### Statistical analysis

Data are shown as mean ± SEM. Statistical analyses were compared using the unpaired Student's two-tailed t-test. Analyses were performed using GraphPad Prism version 6.0 (GraphPad Software). Statistically significant differences of P < 0.05, P < 0.01, P < 0.001 and P < 0.0001 were indicated by *, **, *** and ****, respectively.

### Example 1: All anti-PDL1-IFN fusion proteins constructed can maintain both of the IFNs' biological activity and affinity for PDL1

The systemic administration of type I IFNs has a low response rate and produces severe side effects. The targeted antibody with a cytokine has been shown to be an effective strategy for the local delivery of an immunomodulatory molecule. Considering the high expression of PDL1 molecule in liver of CHB patients, targeting IFNs to the liver by using an anti-PDL1 antibody may exert the anti-viral and immunomodulatory effects locally in the liver, and increase the response rates of IFNs and reduce the side effects.

To achieve this purpose, the present invention proposes to construct a fusion protein anti-PDL1-IFN using an anti-PDL1 antibody with IFNα. A fusion protein containing a Fab fragment of the PDL1-binding polypeptide [Fab(PDL1)] and a IFNα was constructed in the form of a heterodimer (heter) or a homodimer (homo), and named as heter Fab or homo Fab. At the same time, a fusion protein containing a single-chain variable fragment of the PDL1-binding polypeptide [scFv(PDL1)] and a IFNα was constructed in the form of a heterodimer or a homodimer, and named as heter scFv or homo scFv (Figure 1a), and its SDS-PAGE gel image was shown (Figure 1b). Control fusion proteins IFNα-Fc, anti-PDL1 (Fab) and anti-PDL1 (scFv) were also constructed (Figure 1c), and their SDS-PAGE gel images were shown (Figure 1d). Using the Heter Fab heterodimer as an example, the present inventors constructed Anti-PDL1-IFNα (no ADCC) by replacing the Fc region therein with the Fc region in the form of no ADCC, and its SDS-PAGE gel image was shown (Figure 1e). *In vitro* anti-viral activities of the four anti-PDL1-IFN fusion proteins were firstly evaluated, and it was found that all of the four anti-PDL1-IFN fusion proteins effectively inhibited the vesicular stomatitis virus-green fluorescent protein (VSV-GFP) from infecting the L929 cells (Figures. 2a, c), as well as effectively inhibited the replication of VSV-GFP (Figure 2b). This indicates that IFN in the four anti-PDL1-IFN fusion proteins has a good biological activity. Subsequently, the affinities of the four anti-PDL1-IFN fusion proteins and anti-PDL1(Fab) to PDL1 were evaluated using Biolayer interferometry (BLI), and it was found that all of the four anti-PDL1-IFN fusion proteins were able to bind to PDL1, and the heter Fab or homo Fab fusion protein had a similar affinity to PDL1 to that of anti-PDL1(Fab) to PDL1, which was higher than that of the heter scFv or homo scFv fusion protein to PDL1 (Figures 3a-f).

In summary, these data showed that the anti-PDL1-IFN fusion proteins in the form of heterodimers or homodimers were effective in inhibiting the VSV-GFP infection and replication, indicating that all of these fusion proteins maintained the biological activity of IFN. At the same time, all of these fusion proteins maintained the binding abilities to PDL1, with heter Fab or homo Fab having a higher affinity to PDL1 than heter scFv or homo scFv.

### Example 2: The heter Fab or heter scFv fusion protein can deliver IFNs to the liver tissue and have a better stability in the blood.

IFNs can induce the expression of the immunosuppressive molecule PDL1 in addition to their anti-viral and immunomodulatory effects. The binding of PD1 to PDL1 can transmit an inhibitory signal and reduce the activity of immune cells, thereby suppressing a response of the immune cells to HBV. In order to overcome this counteracting effect and achieve a mutual promotion of the immune (re)activation in liver, the fusion protein anti-PDL1-IFN constructed in the present invention can be targeted to the liver by using the highly expressed PDL1 in liver tissues and up-regulate the expression of PDL1 in liver tissues, which results in a further accumulation of the anti-PDL1-IFN fusion protein in liver.

To test this hypothesis, *in vivo* kinetics of the four anti-PDL1-IFN fusion proteins in the HBV carrier mice were firstly detected. Equimolar amounts (0.1687 nmol/mouse) of the four anti-PDL1-IFN fusion proteins or IFNα-Fc fusion protein were injected intravenously into the HBV carrier mice (5 groups in total, with 3 mice each group). After 3 hours of injection, the samples were taken to detect the distribution of the fusion protein in each tissue. It was found that the amount of the heter Fab or heter scFv fusion protein accumulated in liver was significantly higher than that of IFNα-Fc, and significantly higher than that of homo Fab or homo scFv (Figure 4a). The results of 72 hour tissue distribution showed that the amount of the heter Fab or heter scFv fusion protein accumulated in liver was also significantly higher than that of IFNα-Fc, and also significantly higher than that of homo Fab or homo scFv (Figure 4b). The blood concentration of the heter Fab or heter scFv fusion protein was found to be higher than that of the fusion proteins in other forms in the first few hours after the intravenous injection of equimolar amounts (0.1687 nmol/mouse) of the four anti-PDL1-IFN fusion proteins or IFNα-Fc fusion protein by detecting the blood concentrations (Figure 4c).

In summary, the amount of the heter Fab or heter scFv fusion protein accumulated in liver was significantly higher than that of homo Fab, homo scFv, or IFNα-Fc. This significant difference was maintained for at least 72 hours, suggesting that the heter Fab or heter scFv fusion protein had a better effect of targeting the liver. Meanwhile, the blood concentration of the heter Fab or heter scFv fusion protein was higher than that of homo Fab, homo scFv or IFNα-Fc in the first few hours after injection, suggesting that the heter Fab or heter scFv fusion protein had a better stability in the blood.

### Example 3: The inhibitory effect of the heter Fab or heter scFv fusion protein on HBV was better than that of homo Fab or homo scFv in vivo.

Given that the four anti-PDL1-IFN fusion proteins had effective inhibitory activities against VSV-GFP *in vitro,* their anti-viral activities in the HBV carrier mice were further investigated *in vivo.* Firstly, to analyze whether the inhibitory activity of the heter Fab fusion protein against HBV *in vivo* was in a dose-dependent manner, five concentration gradients (5 µg, 10 µg, 20 µg, 40 µg or 80 µg/mouse per time, respectively) were tested by the intravenous injection, with a total of two injections at an interval of 3 days (Figure 5a). It was found that the inhibitory activity of the heter Fab fusion protein against HBV in the HBV carrier mice was in a dose-dependent manner (Figures. 5b, c), and it was also shown that the body weight of the mice decreased significantly with the increase of the dose, indicating that the toxicity increased with the increase of the dose (Figure 5d). All of the five doses used in the experiment were able to reduce the levels of HBV-DNA in serum below the detection limit on day 7, indicating that HBV-DNA in serum was more sensitive to IFN (Figure 5e). Combining the anti-viral activity and *in vivo* toxicity, the dose of 20 µg/mouse per time was found to be less toxic (the body weight of mice decreased within 4%, and recovered after a mild decrease) (Figure 5d) and have a higher inhibitory activity against HBV (The level of HBsAg in serum decreased by nearly 2log₁₀ on average at day 7) (Figure 5b). That is, the optimal *in vivo* dose of the heter Fab fusion protein was 20 µg/mouse per time (0.1687 nmol/mouse per time) and was used as the dose for the subsequent experiments. The inhibitory activities of the four anti-PDL1-IFN fusion proteins against HBV were subsequently compared *in vivo,* wherein the dose of heter Fab was 20 µg/mouse per time (0.1687 nmol/mouse per time), and the doses of the other three fusion proteins were calculated according to the equimolar amounts of IFNα and anti-PDL1. Each fusion protein was injected intravenously twice at an interval of 3 days (Figure 6a). The experimental results showed that the inhibitory effect of the heter Fab or heter scFv fusion protein on HBV was better than that of the homo Fab or homo scFv fusion protein (Figures 6b, c), while the level of HBV-DNA in serum after the treatment with heter Fab fell below the detection limit on day 7 and recovered more slowly (Figure 6d).

In summary, the inhibitory activity of the heter Fab or heter scFv fusion protein against HBV is higher that of homo Fab or homo scFv *in vivo,* which is consistent with the fact that the degree of the heter Fab or heter scFv fusion protein accumulated in liver was significantly higher than that of homo Fab or homo scFv.

### Example 4: The heter Fab fusion protein inhibits HBV in vivo with the synergistic and positive feedback effects.

The HBV carrier mice were injected intravenously with the fusion protein on days 1 and 4 (Figure 7a). The experimental results showed that the inhibitory effect of the heter Fab fusion protein on HBV *in vivo* was better than that of IFNα-Fc or anti-PDL1(Fab) alone, or a physical mixture of IFNα-Fc and anti-PDL1(Fab). The heter Fab fusion protein caused a significant decrease in the serum levels of HBsAg and HBeAg in the HBV carrier mice on day 7 (Figure 7b, c). In addition, the serum levels of HBV-DNA in the IFNα-Fc group, the IFNα-Fc and anti-PDL1(Fab) physical mixing group, and the heter Fab group all decreased significantly on day 7, but the serum level of HBV-DNA in the heter Fab group recovered slower, suggesting that the effect of the heter Fab fusion protein lasted for a longer period of time (Figure 7d).

Since type I interferons were able to induce the expression of PDL1, the expression of PDL1 in liver after the treatment with heter Fab was detected. The results showed that heter Fab was able to significantly increase the expression of PDL1 on CD45⁻ and CD45⁺ cells in liver. The increase in the expression of PDL1 further promoted the accumulation of heter Fab in liver (Figures 7e, f).

In summary, liver targeting is essential for the HBV inhibition by the fusion proteins because the mixture of IFNα-Fc and anti-PDL1 does not produce a synergistic effect as that of the heter Fab fusion protein for the HBV inhibition *in vivo.* Meanwhile, heter Fab has a positive feedback effect that further enhances the accumulation of heter Fab in liver for a better HBV inhibition.

### Example 5: The Heter Fab fusion protein can enhance the function of DC cells, thereby promoting the function of HBsAg-specific T cells and breaking an immune tolerance.

Type I interferons play a key role in promoting the DC cross-presentation to activate T cells. Therefore, whether the administration of heter Fab can enhance the function of DC cells was investigated. HBV carrier mice were treated with a single dose of heter Fab (0.1687 nmol/mouse) and after 3 days, the DC cells in liver and spleen were analyzed by flow cytometry (Figure 8a). The experimental results showed that the treatment with heter Fab resulted in a significant increase in the expression of CD86, CD80 and MHCI on the DC cells in liver and spleen compared to the treatments with other fusion proteins (Figures 8b, c and Figures 9a-d). The up-regulation of these molecules facilitated an activation of T cells by the DC cells. Indeed, heter Fab was able to activate the function of T cells in the HBV carrier mice, and the experiment results from flow cytometry and ELISPOT showed a significant increase in the HBsAg-specific IFN-γ⁺CD4⁺ and CD8⁺ T cells in liver and spleen (Figures 8d, e and Figures 10a-c). To test whether the specific T cells activated by the treatment of the heter Fab fusion protein can exert an inhibitory effect on HBV, T cells in the HBV carrier mice were eliminated before and during the administration of heter Fab. The results showed a significant reduction in the anti-viral effect of heter Fab fusion protein (Figure 8f), and the similar results were obtained using the Rag1^{-/-} HBV carrier mice (Figure 8g). These data suggested that the heter Fab fusion protein-activated T cells were able to exert an inhibitory effect on HBV. In addition, whether the heter Fab fusion protein can enhance the antigen uptake ability of DC cells was detected, and the experimental results showed that the heter Fab fusion protein was able to enhance the uptake of HBsAg-FITC antigen by the DC2.4 cells compared with the treatment with hIgG or IFNα-Fc (Figure 8h). In conclusion, the above data suggest that the heter Fab fusion protein promotes the maturation and antigen-presenting ability of the DC cells, thereby enhancing the function of HBV-specific T cells.

### Example 6: The heter scFv fusion protein in combination with the HBsAg/aluminium adjuvant immunization achieves a functional cure in some HBV carrier mice.

Although the heter Fab or heter scFv fusion protein inhibited HBVs *in vivo,* with a significant decrease in the serum levels of HBsAg, HBeAg and HBV-DNA, this was only sustained for a short period of time (about 14 days), after which it returned to the initial level. This indicates that the treatment with the heter Fab or heter scFv fusion protein alone did not result in a functional cure of the HBV carrier mice. High viral and antigenic loads caused an immune-tolerance to HBV. The present inventors attempted to use the heter Fab or heter scFv fusion protein to transiently remove HBsAg and HBV-DNA from serum, as well as to enhance the functions of DC cells and specific T cells, creating a "window period" to break the immune tolerance to HBV, and then an active immunization using a vaccine was used in combination with the fusion protein to activate the host cellular and humoral immunity and achieve a functional cure for CHB. The immunization using the HBsAg (ayw) antigen alone, the immunization using the anti-PDL1 antibody in combination with the HBsAg (ayw) antigen, the immunization using the IFNα-Fc fusion protein in combination with the HBsAg (ayw) antigen and the immunization using the heter scFv fusion protein in combination with the HBsAg (ayw) antigen were compared, wherein an aluminium adjuvant was used for all immunizations. The fusion protein was injected intravenously twice (at an interval of 3 days) and HBsAg was immunized subcutaneously for four times (at an interval of 7 days) (Figure 11a). The experimental results showed that the immunization using the heter scFv fusion protein in combination with the HBsAg (ayw) antigen was the most effective (Figures 11b, c), with one out of three HBV carrier mice achieving a functional cure, *i.e.,* a serum HBsAg was cleared and the anti-HBsAg antibodies were produced (Fig. 11d, e), and the level of HBV-DNA in serum was close to the detection limit (Figure 11f). In contrast, none of the other treatments resulted in a functional cure in the HBV carrier mice (Figures 11d-f).

In summary, the treatment regimen using the heter scFv fusion protein in combination with the HBsAg/aluminium adjuvant immunization was able to achieve a functional cure of mouse in some HBV carrier mice.

### Example 7: The heter scFv fusion protein in combination with HBsAg/CpG immunization achieves a functional cure in most HBV carrier mice.

The results of Example 6 showed that the heter scFv in combination with the active immunization of vaccine (using an aluminium adjuvant) achieved functional cure in some HBV carrier mice. The present inventors tried to see whether the therapeutic effects can be enhanced by improving the treatment regimen. Firstly, the aluminium immune adjuvant in Example 6 was replaced with CpG adjuvant to promote the humoral and cellular immunity of the organism for a better clearance of HBV. The treatment strategy adopted was shown in Figure 12a, in which the fusion protein was injected intravenously twice (at an interval of 3 days) and the HBsAg(ayw)/CpG was immunized subcutaneously for four times (at an interval of 7 days). The experimental results showed that the treatment regimen using the heter scFv fusion protein in combination with the active immunization of HBsAg(ayw)/CpG resulted in a functional cure in most HBV carrier mice, wherein 3 out of 5 HBV carrier mice achieved a functional cure, increasing the percentage of cure to 60% (Figures 12b-f). The amounts of HBsAg antigen in livers of mice were measured at the end of the experiment (day 104), and it was found that the amount of HBsAg antigen in liver of the combined treatment group (heter scFv+HBsAg/CpG) was significantly decreased compared with the other three groups (Figure 12g). Meanwhile, the HBV-specific T-cells and B-cells in spleens of mice were detected at the end of the experiment (day 104), and the results showed that both HBsAg-specific T-cells and B-cells were significantly elevated in the combined treatment group (Figures 12h, i), suggesting that the combined treatment group induced both cellular immunity and humoral immunity, resulting in a functional cure in most HBV carrier mice.

In summary, the therapeutic effect of CpG adjuvant was superior to that of the aluminium adjuvant, and the CpG adjuvant increased the proportion of functional cure. The treatment regimen using the heter scFv fusion protein in combination with the immunization of HBsAg(ayw)/CpG induced the specific cellular and humoral immunity, and achieved a functional cure in most HBV carrier mice as well as a significant decrease in the level of HBsAg in liver, suggesting that the combined treatment group not only reduced the viral load of HBV in serum, but also reduced the viral load of HBV in liver.

### Example 8: The heter scFv fusion protein in combination with a commercial prophylactic hepatitis B vaccine achieves a functional cure in some HBV carrier mice.

In Example 7, the combined treatment group was able to achieve a functional cure in most HBV carrier mice, and whether the treatment can be achieved using the heter scFv fusion protein directly in combination with a commercial hepatitis B prophylactic vaccine (e.g., EngerixB, with a serotype of type adw) was investigated. The heter scFv fusion protein in combination with the immunize of EngerixB was used to treat the HBV carrier mice (Figure 13a), and it was found that the serum HBsAg levels in mice in the combined treatment group showed a decreasing trend (Figure 13b), while the anti-HBsAg antibodies were produced and maintained for a longer period of time, and the anti-HBsAg antibodies in serum were detectable on day 174 (Figure 13c). Functional cure (40%) was achieved in 2 of 5 HBV carrier mice in the combined treatment group (Figures 13d-f).

In summary, the use of the heter scFv fusion protein in combination with the commercial prophylactic hepatitis B vaccines also achieved a functional cure in some HBV carrier mice, which made the clinical transformation of the fusion protein more convenient.

### Example 9: The immunization using the heter Fab fusion protein in combination with HBsAg/CpG achieves a functional cure in most HBV carrier mice.

The same treatment strategy was used as that in Example 7, in which the fusion protein was injected intravenously twice (at an interval of 3 days) and the HBsAg(ayw)/CpG immunization was performed for four times (at an interval of 7 days) (Figure 14a). The experimental results showed a decreasing trend of the serum HBsAg levels in the HBV carrier mice in the combined treatment group (Figure 14b), and accordingly the level of anti-HBsAg antibodies in the serum of the combined treatment group was the highest and also maintained for the longest time (Figure 14c). At the termination of the experiment, the serum HBsAg levels of 3 mice in the combined treatment group (5 mice in total) turned into being negative (Figure 14d), and accordingly the levels of anti-HBsAg antibodies in the serum of three mice were maintained at high levels (Figure 14e) and the levels of HBV-DNA in serum were close to or below the detection limit (Figure 14f). That is, three HBV-carrier mice in the combined treatment group had achieved a functional cure. Sera from these 3 mice with high antibody levels were injected intravenously into HBV carrier mice without any treatment (100 µL per mouse). The sera from the 3 mice with higher antibody levels in the combined treatment group were able to neutralize HBV *in vivo* compared with the sera from mice in other groups (Figures 14g, h). Meanwhile, the *in vitro* inhibition of HBV infection experiments showed that the anti-HBsAg antibody-containing sera produced by the functionally cured mice in the combined treatment group also inhibited the infection of cells by HBV *in vitro* (Figures 14i, j).

In summary, the treatment using the heter Fab fusion protein in combination with immunization of HBsAg(ayw)/CpG resulted in a functional cure of 60% of HBV carrier mice. Anti-HBsAg antibody level in serum of the combined treatment group reached the highest level at about day 60, and then gradually declined but were maintained for a longer period of time. The produced anti-HBsAg antibodies can neutralize HBV in the body and protect the organism from HBV re-infection.

### Example 10: A mixture of the Heter Fab fusion protein and a commercial prophylactic hepatitis B vaccine can be used as a therapeutic vaccine to produce the anti-HBsAg antibodies and reduce the level of HBsAg in serum.

The therapeutic strategy used was similar to that in Example 8, in which the HBV carrier mice were immunized subcutaneously using a mixture of the Heter Fab fusion protein and a commercial prophylactic hepatitis B vaccine for a total of 3 times at 1-week interval (Figure 15A). Mouse serum was collected, and the levels of anti-HBsAg and HBsAg in serum were detected. The mixture of Heter Fab fusion protein and commercial prophylactic hepatitis B vaccine induced a stronger antibody response compared to the commercial prophylactic hepatitis B vaccine alone (Figure 15B). A significant decrease in the level of HBsAg occurred in the mixed immunization group compared to the vaccine-alone immunization group (Figure 15C), suggesting that the mixture of Heter Fab fusion protein and commercial prophylactic hepatitis B vaccine was helpful to break the immune tolerance to HBsAg.

In summary, the mixture of Heter Fab fusion protein and commercial prophylactic hepatitis B vaccine had a good therapeutic effect in the HBV carrier mice, inducing the production of anti-HBsAg antibodies and decreasing the level of HBsAg in serum.

### Example 11: A targeted interferon anti-PDL1-IFNα as an immune adjuvant promotes the HBV carrier mice to overcome an immune tolerance to the rHBsAg vaccine.

Equimolar amounts (0.01687 nmol, calculated on the basis of a single subunit) of anti-PDL1, IFNα-Fc, anti-PDL1+IFNα-Fc (anti-PDL1 mixed with IFNα-Fc), and anti-PDL1-IFNα were injected subcutaneously alone or mixed with 1 µg of the rHBsAg vaccine to immunize the HBV carrier mice (Figure 16a). Changes in the levels of HBsAg and anti-HBsAg in serum were detected by ELISA. The experimental results showed that only the mixed immunization with anti-PDL1-IFNα and rHBsAg vaccine produced a strong anti-HBsAg antibody response and a decrease in the level of HBsAg in serum, while the other groups were unable to produce an antibody response and did not have a decrease in the level of HBsAg in serum (Figures 16b-e). In addition, neither ALT nor AST was elevated after the mixed subcutaneous immunization (Figure 16f).

In summary, such targeted interferon anti-PDL1-IFNα acting as an immune adjuvant can effectively promote the rHBsAg vaccine immunization with a good safety.

### Example 12: Mixed immunization of anti-PDL1-IFNα and rHBsAg vaccine can induce a strong antibody response.

The HBV carrier mice were immunized subcutaneously with a mixture of 2 µg or 10 µg of anti-PDL1-IFNα and 1 µg of rHBsAg vaccine, and the mixed immunization at the same dose was given 7 days after the initial immunization and the booster immunizations with the rHBsAg vaccine alone were given 3 weeks and 5 weeks after the initial immunization. Changes in the levels of HBsAg and anti-HBsAg in serum were detected by ELISA. The experimental results showed that there was a significant enhancement of the anti-HBsAg antibody response and a significant decrease in the level of HBsAg in the mixed immunization group of anti-PDL1-IFNα and rHBsAg vaccine (Figure 17a). In addition, the HBsAg-specific B and T cell responses were also significantly enhanced in the mixed immunization group (Figures 17b-d).

In summary, anti-PDL1-IFNα as an immune adjuvant can significantly enhance the levels of anti-HBsAg antibody response and HBsAg-specific T-cell response, and can clear the HBsAg antigen in HBV carrier mice.

### Example 13: Anti-PDL1-IFNα as an immune adjuvant can effectively enhance the immunizations against the receptor-binding region (RBD) antigen of SARS-CoV-2, the influenza virus HA1 antigen and the OVA antigen.

Anti-PDL1-IFNα (2 µg/mouse) was mixed with the RBD antigen (3.4 µg/mouse) to immunize mice subcutaneously twice in total at an interval of 14 days. 28 days after the initial immunization, the serum from the immunized mice was collected and the level of anti-RBD antibody in the serum was detected by ELISA (Figure 18a). Mice were immunized subcutaneously with a mixture of anti-PDL1-IFNα (2 µg/mouse) and HA1 antigen (2 µg/mouse). 10 days after the initial immunization, the serum from the immunized mice was collected and the level of anti-HA1 antibody in the serum was detected by ELISA (Figure 18b). Mice were immunized subcutaneously with a mixture of anti-PDL1-IFNα (2 µg/mouse) and OVA antigen (2 µg/mouse). 10 days after the initial immunization, the serum from the immunized mice was collected and the level of anti-OVA antibody in the serum was detected by ELISA (Figure 18c). The experimental results showed that the corresponding antibody levels in the serum of mixed subcutaneous immunization were significantly higher than that in the serum of antigen-alone immunization.

In summary, anti-PDL1-IFNα as an immune adjuvant can promote the immunizations against non-hepatitis B antigens, such as the SARS-CoV-2 receptor-binding region (RBD) antigen, the influenza virus HA1 antigen and the OVA antigen, and significantly increase the antibody levels, thereby significantly enhancing the immune function of the vaccine.

### Example 14: Anti-PDL1-IFNα (human) fusion protein in combination with the rHBsAg vaccine immunization achieves a decrease in the serum HBsAg levels in HBV carrier mice.

The HBV carrier mice were injected intravenously with PBS or anti-PDL1-IFNα (human) fusion protein (20 µg/mouse) on days 1 and 4 (2 groups each, 4 groups in total), respectively. Among them, 1 group of mice injected with PBS and 1 group of mice injected with the anti-PDL1-IFNα (human) fusion protein (2 groups in total) were respectively immunized subcutaneously with rHBsAg (1 µg/mouse) for 4 times on days 4, 11, 18 and 25 *(i.e.,* the groups of "anti-PDL1-IFNα (human) + rHBsAg" and "rHBsAg alone + PBS"). The levels of HBsAg in serum at each time point were detected by ELISA. The experimental results showed that the intravenous injection of anti-PDL1-IFNα (human) in combination with subcutaneous administration of rHBsAg was the most effective, achieving a decrease in the level of HBsAg in serum (Figure 19).

In summary, the replacement of interferon in the fusion protein from mouse IFNα4 to human IFNα2 (Q124R) also achieved the same effect of promoting an rHBsAg vaccine immunization.

### Example 15: Anti-PDL1-IFNα (human) as a targeted immune adjuvant can effectively promote an rHBsAg vaccine immunization.

Anti-PDL1-IFNα (human) (2 µg/mouse) was mixed with rHBsAg (1 µg/mouse) to immunize the HBV carrier mice subcutaneously, and the level of HBsAg in the serum at each time point was detected by ELISA. The experimental results showed that the mixed subcutaneous immunization and treatment with anti-PDL1-IFNα (human) and rHBsAg vaccine achieved a decrease in the level of HBsAg in serum (Figure 20).

In summary, anti-PDL1-IFNα (human) also functions as an immune adjuvant, and the mixed subcutaneous immunization with rHBsAg can overcome an immune tolerance and promote an effect of vaccine immunization.

The above descriptions are only the preferred examples of the present invention, but cannot be used to limit the present invention, and any modification, equivalent substitution, improvement, etc. made within the spirit and principles of the present invention shall be included in the protection scope of the present invention.

### Sequence Listing

SEQ ID NO.1: the amino acid sequence of mIFNα4-Fc (hole) in the heter Fab or heter scFv heterodimer wherein the underlined bold sequence is a linker sequence) 
CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKESGGGGSGGGGSGGG
GSGGGGRTDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAP
IEKTISKAKGQPREPQVYTLPPCRDELTKNQVSLWCLVKGFYPSDIAVEWESNGQPENNYK
TTPPVLDSDGSFFLYSALTVDKSRWQQGNVFSCSVMHEALHNHYTQKSLSLSPGK
SEQ ID NO.2: the nucleotide sequence encoding mIFNα4-Fc (hole) in the heter Fab or heter scFv heterodimer (wherein the underlined bold sequence is a linker sequence) 
tgtgacctgcctcacacttataacctcgggaacaagagggccttgacagtcctggaagaaatgagaagactcccccctctttcctgcct
gaaggacaggaaggattttggattccccttggagaaggtggataaccaacagatccagaaggctcaagccatccttgtgctaagagatcttac
ccagcagattttgaacctcttcacatcaaaagacttgtctgctacttggaatgcaactctactagactcattctgcaatgacctccatcagcagctc
aatgacctcaaagcctgtgtgatgcaggaacctcctctgacccaggaagactccctgctggctgtgaggacatacttccacaggatcactgtgt
acctgagaaagaagaaacacagcctctgtgcctgggaggtgatcagagcagaagtctggagagccctctcttcctcaaccaacttgctggca
agactgagtgaggagaaggagagtggcggtggtggctccggcggtggtggctccggcggtggtggctccggcggtggtggccgtacg
gacaaaactcacacatgcccaccgtgcccagctccggaactcctgggcggaccgtcagtcttcctcttccccccaaaacccaaggacaccct
catgatctcccggacccctgaggtcacatgcgtggtggtggacgtgagccacgaagaccctgaggtcaagttcaactggtacgtggacggc
gtggaggtgcataatgccaagacaaagccgcgggaggagcagtacaacagcacgtaccgtgtggtcagcgtcctcaccgtcctgcaccag
gactggctgaatggcaaggagtacaagtgcaaggtctccaacaaagccctcccagcccccatcgagaaaaccatctccaaagccaaaggg
cagccccgagaaccacaggtgtacaccctgcccccatgtcgggatgagctgaccaagaaccaggtcagcctgtggtgcctggtcaaaggct
tctatcccagcgacatcgccgtggagtgggagagcaatgggcagccggagaacaactacaagaccacgcctcccgtgttggactccgacg
gctccttcttcctctacagcgcgctcaccgtggacaagagcaggtggcagcaggggaacgtcttctcatgctccgtgatgcatgaggctctgc
acaaccactacacgcagaagagcctctccctgtctccgggtaaa
SEQ ID NO.3: the amino acid sequence of the anti-PDL1 light chain in the heter Fab heterodimer, the homo Fab homodimer or the anti-PDL1 (Fab) complete antibody 
DIQMTQSPSSLSASVGDRVTITCRASQDVSTAVAWYQQKPGKAPKLLIYSASFLYSGV
PSRFSGSGSGTDFTLTISSLQPEDFATYYCQQYLYHPATFGQGTKVEIKRRTVAAPSVFIFPPS
 
DEQLKSGTASVVCLLNNFYPREAKVQWKVDNALQSGNSQESVTEQDSKDSTYSLSSTLTL
SKADYEKHKVYACEVTHQGLSSPVTKSFNRGEC
SEQ ID NO.4: the nucleotide sequence encoding the anti-PDL1 light chain in the heter Fab heterodimer, the homo Fab homodimer or the anti-PDL1 (Fab) complete antibody 
gacatccagatgacccagagccccagcagcctgagcgccagcgtgggcgacagagtgaccatcacctgcagagccagccaggac
gtgagcaccgccgtggcctggtaccagcagaagcccggcaaggcccccaagctgctgatctacagcgccagcttcctgtacagcggcgtg
cccagcagattcagcggcagcggcagcggcaccgacttcaccctgaccatcagcagcctgcagcccgaggacttcgccacctactactgc
cagcagtacctgtaccaccccgccaccttcggccagggcaccaaggtggagatcaagagacgtacggtggctgcaccatctgtcttcatctt
cccgccatctgatgagcagttgaaatctggtaccgctagcgttgtgtgcctgctgaataacttttatccacgggaggctaaggtgcagtggaaa
gtggacaatgccctccagagcggaaatagccaagagtccgttaccgaacaggactctaaagactctacatactccctgtcctccacactgacc
ctctccaaggccgactatgagaaacacaaggtttacgcatgcgaggtcacacaccagggactctcctctcccgtgaccaagagcttcaaccg
gggagaatgc
SEQ ID NO.5: the amino acid sequence of anti-PDL1 heavy chain Fab fragment-Fc (knob) in the heter Fab heterodimer 
EVQLVESGGGLVQPGGSLRLSCAASGFTFSDSWIHWVRQAPGKGLEWVAWISPYGGS
TYYADSVKGRFTISADTSKNTAYLQMNSLRAEDTAVYYCARRHWPGGFDYWGQGTLVT
VSAASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVCTLPPSRD
ELTKNQVSLSCAVKGFYPSDIAVEWESNGQPENNYKTTPPVLDSDGSFKLVSKLTVDKSR
WQQGNVFSCSVMHEALHNHYTQKSLSLSPGK
SEQ ID NO.6: the nucleotide sequence encoding anti-PDL1 heavy chain Fab fragment-Fc (knob) in the heter Fab heterodimer 
gaggtgcagctggtggagagcggcggcggcctggtgcagcccggcggcagcctgagactgagctgcgccgccagcggcttcac
cttcagcgacagctggatccactgggtgagacaggcccccggcaagggcctggagtgggtggcctggatcagcccctacggcggcagca
cctactacgccgacagcgtgaagggcagattcaccatcagcgccgacaccagcaagaacaccgcctacctgcagatgaacagcctgagag
ccgaggacaccgccgtgtactactgcgccagaagacactggcccggcggcttcgactactggggccagggcaccctggtgaccgtgagc
gccgccagcactaagggcccctctgtgtttccactcgccccttctagcaaaagcacttccggaggaactgccgctctgggctgtctggtgaaa
gattacttccccgaaccagtcactgtgtcatggaactctggagcactgacatctggagttcacacctttcctgctgtgctgcagagttctggactg
tactccctgtcatctgtggtcaccgtgccatcttcatctctggggacccagacctacatctgtaacgtgaaccacaaaccctccaacacaaaagt
cgacaaacgagtcgaaccaaaatcttgtgacaaaacccacacatgcccaccttgtcccgcccctgaactcctgggcggaccgtcagtcttcct
cttccccccaaaacccaaggacaccctcatgatctcccggacccctgaggtcacatgcgtggtggtggacgtgagccacgaagaccctgag
gtcaagttcaactggtacgtggacggcgtggaggtgcataatgccaagacaaagccgcgggaggagcagtacaacagcacgtaccgtgtg
gtcagcgtcctcaccgtcctgcaccaggactggctgaatggcaaggagtacaagtgcaaggtctccaacaaagccctcccagcccccatcg
agaaaaccatctccaaagccaaagggcagccccgagaaccacaggtgtgtaccctgcccccatcccgggatgagctgaccaagaaccag
gtcagcctgagttgcgcggtcaaaggcttctatcccagcgacatcgccgtggagtgggagagcaatgggcagccggagaacaactacaag
accacgcctcccgtgttggactccgacggctccttcaagctcgtcagcaagctcaccgtggacaagagcaggtggcagcaggggaacgtct
tctcatgctccgtgatgcatgaggctctgcacaaccactacacgcagaagagcctctccctgtctccgggtaaa
SEQ ID NO.7: the amino acid sequence of mIFNα4- anti-PDL1 heavy chain Fab fragment-Fc (hIgG1-Fc, WT) in the homo Fab homodimer (wherein the underlined bold sequence is a linker sequence) 
CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKESGGGGSGGGGSGGG
GSGGGGRTEVQLVESGGGLVQPGGSLRLSCAASGFTFSDSWIHWVRQAPGKGLEWVAWI
SPYGGSTYYADSVKGRFTISADTSKNTAYLQMNSLRAEDTAVYYCARRHWPGGFDYWGQ
GTLVTVSAASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKSCFEEPKSCDKTHT
CPPCPAPELLGGPSVFLFPPKPKDQLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFLY
SKLTVDKSRWQQGNVFSCSVLHEALHNHYTQKSLSLSPGK
SEQ ID NO.8: the nucleotide sequence encoding the mIFNα4-anti-PDL1 heavy chain Fab fragment-Fc (hIgG1-Fc, WT) in the homo Fab homodimer (wherein the underlined bold sequence is a linker sequence) 
tgtgacctgcctcacacttataacctcgggaacaagagggccttgacagtcctggaagaaatgagaagactcccccctctttcctgcct
gaaggacaggaaggattttggattccccttggagaaggtggataaccaacagatccagaaggctcaagccatccttgtgctaagagatcttac
ccagcagattttgaacctcttcacatcaaaagacttgtctgctacttggaatgcaactctactagactcattctgcaatgacctccatcagcagctc
aatgacctcaaagcctgtgtgatgcaggaacctcctctgacccaggaagactccctgctggctgtgaggacatacttccacaggatcactgtgt
acctgagaaagaagaaacacagcctctgtgcctgggaggtgatcagagcagaagtctggagagccctctcttcctcaaccaacttgctggca
agactgagtgaggagaaggagagtggcggtggtggctccggcggtggtggctccggcggtggtggctccggcggtggtggccgtacg
gaggtgcagctggtggagagcggcggcggcctggtgcagcccggcggcagcctgagactgagctgcgccgccagcggcttcaccttca
gcgacagctggatccactgggtgagacaggcccccggcaagggcctggagtgggtggcctggatcagcccctacggcggcagcacctac
tacgccgacagcgtgaagggcagattcaccatcagcgccgacaccagcaagaacaccgcctacctgcagatgaacagcctgagagccga
ggacaccgccgtgtactactgcgccagaagacactggcccggcggcttcgactactggggccagggcaccctggtgaccgtgagcgccg
ccagcactaaggggccctctgtgtttccactcgccccttctagcaaaagcacttccggaggaactgccgctctgggctgtctggtgaaagatta
cttccccgaaccagtcactgtgtcatggaactctggagcactgacatctggagttcacacctttcctgctgtgctgcagagttctggactgtactc
cctgtcatctgtggtcaccgtgccatcttcatctctggggacccagacctacatctgtaacgtgaaccacaaaccctccaacacaaaagtcgac
aaacgagtcgaaccaaaatcttgtttcgaagagcccaagagctgcgacaagacccacacctgtcccccttgtcctgcccctgagctgctggg
cggacccagcgtgttcctgttccccccaaagcccaaggaccagctgatgatcagccggacccccgaagtgacctgcgtggtggtggacgtg
tcccacgaggaccctgaagtgaagttcaattggtacgtggacggcgtggaggtgcacaacgccaagaccaagccccgggaggaacagta
caacagcacctaccgggtggtgtccgtgctgaccgtgctgcaccaggactggctgaacggcaaagagtacaagtgcaaggtctccaacaa
ggccctgcctgcccccatcgaaaagaccatcagcaaggccaagggccagcccagagaaccccaggtgtacaccctgccccccagcagag
atgagctgaccaagaaccaggtgtccctgacctgcctggtcaagggcttctaccccagcgatatcgccgtggagtgggagagcaacggcca
gcctgagaacaactacaagaccaccccccctgtgctggacagcgatggcagcttcctctacagcaaactgaccgtggacaagagccggtgg
cagcagggcaacgtgttcagctgcagcgtgttgcacgaggccctgcacaaccactacacccagaagtccctgagcctgagccccggcaaa
SEQ ID NO.9: the amino acid sequence of scFv (PDL1)-Fc (knob) in the heter scFv heterodimer (wherein the underlined bold sequence is a linker sequence) 
ELDIQMTQSPSSLSASVGDRVTITCRASQDVSTAVAWYQQKPGKAPKLLIYSASFLYS
GVPSRFSGSGSGTDFTLTISSLQPEDFATYYCQQYLYHPATFGQGTKVEIKRGGGGSGGGG
SGGGGSEVQLVESGGGLVQPGGSLRLSCAASGFTFSDSWIHWVRQAPGKGLEWVAWISP
YGGSTYYADSVKGRFTISADTSKNTAYLQMNSLRAEDTAVYYCARRHWPGGFDYWGQG
TLVTVSARSDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAP
IEKTISKAKGQPREPQVYTLPPCRDELTKNQVSLWCLVKGFYPSDIAVEWESNGQPENNYK
TTPPVLDSDGSFFLYSKLTVDKSRWQQGNVFSCSVMHEALHNHYTQKSLSLSPGK
SEQ ID NO.10: the nucleotide sequence encoding scFv (PDL1)-Fc (knob) in the heter scFv heterodimer (wherein the underlined bold sequence is a linker sequence) 
gagctcgacatccagatgacccagagccccagcagcctgagcgccagcgtgggcgacagagtgaccatcacctgcagagccagc
caggacgtgagcaccgccgtggcctggtaccagcagaagcccggcaaggcccccaagctgctgatctacagcgccagcttcctgtacagc
ggcgtgcccagcagattcagcggcagcggcagcggcaccgacttcaccctgaccatcagcagcctgcagcccgaggacttcgccacctac
tactgccagcagtacctgtaccaccccgccaccttcggccagggcaccaaggtggagatcaagagaggcggcggcggcagcggcggcg
gcggcagcggcggcggcggcagcgaggtgcagctggtggagagcggcggcggcctggtgcagcccggcggcagcctgagactgagc
tgcgccgccagcggcttcaccttcagcgacagctggatccactgggtgagacaggcccccggcaagggcctggagtgggtggcctggatc
agcccctacggcggcagcacctactacgccgacagcgtgaagggcagattcaccatcagcgccgacaccagcaagaacaccgcctacct
gcagatgaacagcctgagagccgaggacaccgccgtgtactactgcgccagaagacactggcccggcggcttcgactactggggccagg
gcaccctggtgaccgtgagcgccagatctgacaagacccacacctgccccccctgccccgcccccgagctgctgggcggccccagcgtgt
tcctgttcccccccaagcccaaggacaccctgatgatcagccgcacccccgaggtgacctgcgtggtggtggacgtgagccacgaggacc
ccgaggtgaagttcaactggtacgtggacggcgtggaggtgcacaacgccaagaccaagccccgcgaggagcagtacaacagcacctac
cgcgtggtgagcgtgctgaccgtgctgcaccaggactggctgaacggcaaggagtacaagtgcaaggtgagcaacaaggccctgcccgc
ccccatcgagaagaccatcagcaaggccaagggccagccccgcgagccccaggtgtacaccctgcccccctgccgcgacgagctgacc
aagaaccaggtgagcctgtggtgcctggtgaagggcttctaccccagcgacatcgccgtggagtgggagagcaacggccagcccgagaa
caactacaagaccaccccccccgtgctggacagcgacggcagcttcttcctgtacagcaagctgaccgtggacaagagccgctggcagca
gggcaacgtgttcagctgcagcgtgatgcacgaggccctgcacaaccactacacccagaagagcctgagcctgagccccggcaagtaa
SEQ ID NO.11: the amino acid sequence of mIFNα4-scFv (PDL1)-Fc (hIgG1-Fc, WT) in the homo scFv homodimer (wherein the underlined bold sequence is a linker sequence) 
CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKESGGGGSGGGGSGGG
GSGGGGRTDIQMTQSPSSLSASVGDRVTITCRASQDVSTAVAWYQQKPGKAPKLLIYSASF
LYSGVPSRFSGSGSGTDFTLTISSLQPEDFATYYCQQYLYHPATFGQGTKVEIKRGGGGSG
 
GGGSGGGGSEVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFDYW
GQGTLVTVSAFEEPKSCDKTHTCPPCPAPELLGGPSVFLFPPKPKDQLMISRTPEVTCVVVD
VSHEDPEVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIAVEWESN
GQPENNYKTTPPVLDSDGSFLYSKLTVDKSRWQQGNVFSCSVLHEALHNHYTQKSLSLSP
GK
SEQ ID NO.12: the nucleotide sequence encoding mIFNα4-scFv (PDL1)-Fc (hIgG1-Fc, WT) in the homo scFv homodimer (wherein the underlined bold sequence is a linker sequence) 
tgtgacctgcctcacacttataacctcgggaacaagagggccttgacagtcctggaagaaatgagaagactcccccctctttcctgcct
gaaggacaggaaggattttggattccccttggagaaggtggataaccaacagatccagaaggctcaagccatccttgtgctaagagatcttac
ccagcagattttgaacctcttcacatcaaaagacttgtctgctacttggaatgcaactctactagactcattctgcaatgacctccatcagcagctc
aatgacctcaaagcctgtgtgatgcaggaacctcctctgacccaggaagactccctgctggctgtgaggacatacttccacaggatcactgtgt
acctgagaaagaagaaacacagcctctgtgcctgggaggtgatcagagcagaagtctggagagccctctcttcctcaaccaacttgctggca
agactgagtgaggagaaggagagtggcggtggtggctccggcggtggtggctccggcggtggtggctccggcggtggtggccgtacg
gacatccagatgacccagagccccagcagcctgagcgccagcgtgggcgacagagtgaccatcacctgcagagccagccaggacgtga
gcaccgccgtggcctggtaccagcagaagcccggcaaggcccccaagctgctgatctacagcgccagcttcctgtacagcggcgtgccca
gcagattcagcggcagcggcagcggcaccgacttcaccctgaccatcagcagcctgcagcccgaggacttcgccacctactactgccagc
agtacctgtaccaccccgccaccttcggccagggcaccaaggtggagatcaagagaggcggcggcggcagcggcggcggcggcagcg
gcggcggcggcagcgaggtgcagctggtggagagcggcggcggcctggtgcagcccggcggcagcctgagactgagctgcgccgcc
agcggcttcaccttcagcgacagctggatccactgggtgagacaggcccccggcaagggcctggagtgggtggcctggatcagcccctac
ggcggcagcacctactacgccgacagcgtgaagggcagattcaccatcagcgccgacaccagcaagaacaccgcctacctgcagatgaa
cagcctgagagccgaggacaccgccgtgtactactgcgccagaagacactggcccggcggcttcgactactggggccagggcaccctgg
tgaccgtgagcgccttcgaagagcccaagagctgcgacaagacccacacctgtcccccttgtcctgcccctgagctgctgggcggacccag
cgtgttcctgttccccccaaagcccaaggaccagctgatgatcagccggacccccgaagtgacctgcgtggtggtggacgtgtcccacgag
gaccctgaagtgaagttcaattggtacgtggacggcgtggaggtgcacaacgccaagaccaagccccgggaggaacagtacaacagcac
ctaccgggtggtgtccgtgctgaccgtgctgcaccaggactggctgaacggcaaagagtacaagtgcaaggtctccaacaaggccctgcct
gcccccatcgaaaagaccatcagcaaggccaagggccagcccagagaaccccaggtgtacaccctgccccccagcagagatgagctgac
caagaaccaggtgtccctgacctgcctggtcaagggcttctaccccagcgatatcgccgtggagtgggagagcaacggccagcctgagaa
caactacaagaccaccccccctgtgctggacagcgatggcagcttcctctacagcaaactgaccgtggacaagagccggtggcagcaggg
caacgtgttcagctgcagcgtgttgcacgaggccctgcacaaccactacacccagaagtccctgagcctgagccccggcaaa
SEQ ID NO.13 : the amino acid sequence of IFNα-Fc (hIgG1-Fc, WT) (wherein the underlined bold sequence is a linker sequence) 
CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKESGGGGSGGGGSGGG
GSGGGGRTEPKSCDKTHTCPPCPAPELLGGPSVFLFPPKPKDQLMISRTPEVTCVVVDVSH
EDPEVKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWESNGQP
ENNYKTTPPVLDSDGSFLYSKLTVDKSRWQQGNVFSCSVLHEALHNHYTQKSLSLSPGK
SEQ ID NO.14: the nucleotide sequence encoding IFNα-Fc (hIgG1-Fc, WT) (wherein the underlined bold sequence is a linker sequence) 
tgtgacctgcctcacacttataacctcgggaacaagagggccttgacagtcctggaagaaatgagaagactcccccctctttcctgcct
gaaggacaggaaggattttggattccccttggagaaggtggataaccaacagatccagaaggctcaagccatccttgtgctaagagatcttac
ccagcagattttgaacctcttcacatcaaaagacttgtctgctacttggaatgcaactctactagactcattctgcaatgacctccatcagcagctc
aatgacctcaaagcctgtgtgatgcaggaacctcctctgacccaggaagactccctgctggctgtgaggacatacttccacaggatcactgtgt
acctgagaaagaagaaacacagcctctgtgcctgggaggtgatcagagcagaagtctggagagccctctcttcctcaaccaacttgctggca
agactgagtgaggagaaggagagtggcggtggtggctccggcggtggtggctccggcggtggtggctccggcggtggtggccgtacg
gagcccaagagctgcgacaagacccacacctgtcccccttgtcctgcccctgagctgctgggcggacccagcgtgttcctgttccccccaaa
gcccaaggaccagctgatgatcagccggacccccgaagtgacctgcgtggtggtggacgtgtcccacgaggaccctgaagtgaagttcaat
tggtacgtggacggcgtggaggtgcacaacgccaagaccaagccccgggaggaacagtacaacagcacctaccgggtggtgtccgtgct
gaccgtgctgcaccaggactggctgaacggcaaagagtacaagtgcaaggtctccaacaaggccctgcctgcccccatcgaaaagaccat
cagcaaggccaagggccagcccagagaaccccaggtgtacaccctgccccccagcagagatgagctgaccaagaaccaggtgtccctga
cctgcctggtcaagggcttctaccccagcgatatcgccgtggagtgggagagcaacggccagcctgagaacaactacaagaccacccccc
ctgtgctggacagcgatggcagcttcctctacagcaaactgaccgtggacaagagccggtggcagcagggcaacgtgttcagctgcagcgt
gttgcacgaggccctgcacaaccactacacccagaagtccctgagcctgagccccggcaaa
SEQ ID NO.15: the amino acid sequence of the heavy chain of the anti-PDL1 (Fab) complete antibody (wherein, Fc is hIgG1-Fc, WT) 
EVQLVESGGGLVQPGGSLRLSCAASGFTFSDSWIHWVRQAPGKGLEWVAWISPYGGS
TYYADSVKGRFTISADTSKNTAYLQMNSLRAEDTAVYYCARRHWPGGFDYWGQGTLVT
VSAASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCRTEPKSCDKTHTCPPCP
APELLGGPSVFLFPPKPKDQLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFLYSKLTV
DKSRWQQGNVFSCSVLHEALHNHYTQKSLSLSPGK
SEQ ID NO.16: the nucleotide sequence encoding the heavy chain of the anti-PDL1 (Fab) complete antibody (wherein, Fc is hIgG1-Fc, WT) 
gaggtgcagctggtggagagcggcggcggcctggtgcagcccggcggcagcctgagactgagctgcgccgccagcggcttcac
cttcagcgacagctggatccactgggtgagacaggcccccggcaagggcctggagtgggtggcctggatcagcccctacggcggcagca
cctactacgccgacagcgtgaagggcagattcaccatcagcgccgacaccagcaagaacaccgcctacctgcagatgaacagcctgagag
ccgaggacaccgccgtgtactactgcgccagaagacactggcccggcggcttcgactactggggccagggcaccctggtgaccgtgagc
gccgccagcactaagggcccctctgtgtttccactcgccccttctagcaaaagcacttccggaggaactgccgctctgggctgtctggtgaaa
gattacttccccgaaccagtcactgtgtcatggaactctggagcactgacatctggagttcacacctttcctgctgtgctgcagagttctggactg
tactccctgtcatctgtggtcaccgtgccatcttcatctctggggacccagacctacatctgtaacgtgaaccacaaaccctccaacacaaaagt
cgacaaacgagtcgaaccaaaatcttgtcgtacggagcccaagagctgcgacaagacccacacctgtcccccttgtcctgcccctgagctgc
tgggcggacccagcgtgttcctgttccccccaaagcccaaggaccagctgatgatcagccggacccccgaagtgacctgcgtggtggtgga
cgtgtcccacgaggaccctgaagtgaagttcaattggtacgtggacggcgtggaggtgcacaacgccaagaccaagccccgggaggaac
agtacaacagcacctaccgggtggtgtccgtgctgaccgtgctgcaccaggactggctgaacggcaaagagtacaagtgcaaggtctccaa
caaggccctgcctgcccccatcgaaaagaccatcagcaaggccaagggccagcccagagaaccccaggtgtacaccctgccccccagca
gagatgagctgaccaagaaccaggtgtccctgacctgcctggtcaagggcttctaccccagcgatatcgccgtggagtgggagagcaacg
gccagcctgagaacaactacaagaccaccccccctgtgctggacagcgatggcagcttcctctacagcaaactgaccgtggacaagagcc
ggtggcagcagggcaacgtgttcagctgcagcgtgttgcacgaggccctgcacaaccactacacccagaagtccctgagcctgagccccg
gcaaa
SEQ ID NO.17: the amino acid sequence of anti-PDL1 (scFv) (wherein, Fc is hIgG1-Fc, WT) (wherein the underlined bold sequence is the linker sequence) 
DIQMTQSPSSLSASVGDRVTITCRASQDVSTAVAWYQQKPGKAPKLLIYSASFLYSGV
PSRFSGSGSGTDFTLTISSLQPEDFATYYCQQYLYHPATFGQGTKVEIKRGGGGSGGGGSG
GGGSEVQLVESGGGLVQPGGSLRLSCAASGFTFSDSWIHWVRQAPGKGLEWVAWISPYG
GSTYYADSVKGRFTISADTSKNTAYLQMNSLRAEDTAVYYCARRHWPGGFDYWGQGTLV
TVSARTEPKSCDKTHTCPPCPAPELLGGPSVFLFPPKPKDQLMISRTPEVTCVVVDVSHEDP
EVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPENN
YKTTPPVLDSDGSFLYSKLTVDKSRWQQGNVFSCSVLHEALHNHYTQKSLSLSPGK
SEQ ID NO.18: the nucleotide sequence encoding anti-PDL1 (scFv) (wherein, Fc is hIgG1-Fc, WT) (wherein the underlined bold sequence is a linker sequence) 
gacatccagatgacccagagccccagcagcctgagcgccagcgtgggcgacagagtgaccatcacctgcagagccagccaggac
gtgagcaccgccgtggcctggtaccagcagaagcccggcaaggcccccaagctgctgatctacagcgccagcttcctgtacagcggcgtg
cccagcagattcagcggcagcggcagcggcaccgacttcaccctgaccatcagcagcctgcagcccgaggacttcgccacctactactgc
cagcagtacctgtaccaccccgccaccttcggccagggcaccaaggtggagatcaagagaggcggcggcggcagcggcggcggcggc
agcggcggcggcggcagcgaggtgcagctggtggagagcggcggcggcctggtgcagcccggcggcagcctgagactgagctgcgc
cgccagcggcttcaccttcagcgacagctggatccactgggtgagacaggcccccggcaagggcctggagtgggtggcctggatcagcc
cctacggcggcagcacctactacgccgacagcgtgaagggcagattcaccatcagcgccgacaccagcaagaacaccgcctacctgcag
atgaacagcctgagagccgaggacaccgccgtgtactactgcgccagaagacactggcccggcggcttcgactactggggccagggcac
cctggtgaccgtgagcgcccgtacggagcccaagagctgcgacaagacccacacctgtcccccttgtcctgcccctgagctgctgggcgg
acccagcgtgttcctgttccccccaaagcccaaggaccagctgatgatcagccggacccccgaagtgacctgcgtggtggtggacgtgtcc
cacgaggaccctgaagtgaagttcaattggtacgtggacggcgtggaggtgcacaacgccaagaccaagccccgggaggaacagtacaa
cagcacctaccgggtggtgtccgtgctgaccgtgctgcaccaggactggctgaacggcaaagagtacaagtgcaaggtctccaacaaggc
cctgcctgcccccatcgaaaagaccatcagcaaggccaagggccagcccagagaaccccaggtgtacaccctgccccccagcagagatg
agctgaccaagaaccaggtgtccctgacctgcctggtcaagggcttctaccccagcgatatcgccgtggagtgggagagcaacggccagc
ctgagaacaactacaagaccaccccccctgtgctggacagcgatggcagcttcctctacagcaaactgaccgtggacaagagccggtggca
gcagggcaacgtgttcagctgcagcgtgttgcacgaggccctgcacaaccactacacccagaagtccctgagcctgagccccggcaaa
SEQ ID NO.19: the amino acid sequence of mIFNα4-Fc (no ADCC) in the anti-PDL1-IFNα heterodimer (wherein the underlined bold sequence is a linker sequence) 
CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKESGGGGSGGGGSGGG
GSGGGGRTDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALG
APIEKTISKAKGQPREPQVYTLPPCRDELTKNQVSLWCLVKGFYPSDIAVEWESNGQPENN
YKTTPPVLDSDGSFFLYSALTVDKSRWQQGNVFSCSVMHEALHNHYTQKSLSLSPGK
SEQ ID NO.20: the nucleotide sequence encoding mIFNα4-Fc (no ADCC) in the anti-PDL1-IFNα heterodimer (wherein the underlined bold sequence is a linker sequence) 
tgtgacctgcctcacacttataacctcgggaacaagagggccttgacagtcctggaagaaatgagaagactcccccctctttcctgcctgaag
gacaggaaggattttggattccccttggagaaggtggataaccaacagatccagaaggctcaagccatccttgtgctaagagatcttacccag
cagattttgaacctcttcacatcaaaagacttgtctgctacttggaatgcaactctactagactcattctgcaatgacctccatcagcagctcaatg
acctcaaagcctgtgtgatgcaggaacctcctctgacccaggaagactccctgctggctgtgaggacatacttccacaggatcactgtgtacct
gagaaagaagaaacacagcctctgtgcctgggaggtgatcagagcagaagtctggagagccctctcttcctcaaccaacttgctggcaaga
ctgagtgaggagaaggagagtggcggtggtggctccggcggtggtggctccggcggtggtggctccggcggtggtggccgtacggac
aaaacccacacatgcccaccttgtcccgcccctgaagccgcaggcggaccgtcagtcttcctcttccccccaaaacccaaggacaccctcat
gatctcccggacccctgaggtcacatgcgtggtggtggacgtgagccacgaagaccctgaggtcaagttcaactggtacgtggacggcgtg
gaggtgcataatgccaagacaaagccgcgggaggagcagtacaacagcacgtaccgtgtggtcagcgtcctcaccgtcctgcaccaggac
tggctgaatggcaaggagtacaagtgcaaggtctccaacaaagccctcggagcccccatcgagaaaaccatctccaaagccaaagggcag
ccccgagaaccacaggtgtacaccctgcccccatgtcgggatgagctgaccaagaaccaggtcagcctgtggtgcctggtcaaaggcttct
atcccagcgacatcgccgtggagtgggagagcaatgggcagccggagaacaactacaagaccacgcctcccgtgttggactccgacggct
ccttcttcctctacagcgcgctcaccgtggacaagagcaggtggcagcaggggaacgtcttctcatgctccgtgatgcatgaggctctgcaca
accactacacgcagaagagcctctccctgtctccgggtaaa
SEQ ID NO.21: the amino acid sequence of the anti-PDL1 heavy chain Fab fragment-Fc (no ADCC) in the anti-PDL1-IFNα heterodimer 
EVQLVESGGGLVQPGGSLRLSCAASGFTFSDSWIHWVRQAPGKGLEWVAWISPYGGS
TYYADSVKGRFTISADTSKNTAYLQMNSLRAEDTAVYYCARRHWPGGFDYWGQGTLVT
VSAASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALGAPIEKTISKAKGQPREPQVCTLPPSR
DELTKNQVSLSCAVKGFYPSDIAVEWESNGQPENNYKTTPPVLDSDGSFKLVSKLTVDKSR
WQQGNVFSCSVMHEALHNHYTQKSLSLSPGK
SEQ ID NO.22: the nucleotide sequence encoding the anti-PDL1 heavy chain Fab fragment-Fc (no ADCC) in the anti-PDL1-IFNα heterodimer 
gaggtgcagctggtggagagcggcggcggcctggtgcagcccggcggcagcctgagactgagctgcgccgccagcggcttcac
cttcagcgacagctggatccactgggtgagacaggcccccggcaagggcctggagtgggtggcctggatcagcccctacggcggcagca
cctactacgccgacagcgtgaagggcagattcaccatcagcgccgacaccagcaagaacaccgcctacctgcagatgaacagcctgagag
ccgaggacaccgccgtgtactactgcgccagaagacactggcccggcggcttcgactactggggccagggcaccctggtgaccgtgagc
gccgccagcactaagggcccctctgtgtttccactcgccccttctagcaaaagcacttccggaggaactgccgctctgggctgtctggtgaaa
gattacttccccgaaccagtcactgtgtcatggaactctggagcactgacatctggagttcacacctttcctgctgtgctgcagagttctggactg
tactccctgtcatctgtggtcaccgtgccatcttcatctctggggacccagacctacatctgtaacgtgaaccacaaaccctccaacacaaaagt
cgacaaacgagtcgaaccaaaatcttgtgacaaaacccacacatgcccaccttgtcccgcccctgaagccgcaggcggaccgtcagtcttcc
tcttccccccaaaacccaaggacaccctcatgatctcccggacccctgaggtcacatgcgtggtggtggacgtgagccacgaagaccctga
ggtcaagttcaactggtacgtggacggcgtggaggtgcataatgccaagacaaagccgcgggaggagcagtacaacagcacgtaccgtgt
ggtcagcgtcctcaccgtcctgcaccaggactggctgaatggcaaggagtacaagtgcaaggtctccaacaaagccctcggagcccccatc
gagaaaaccatctccaaagccaaagggcagccccgagaaccacaggtgtgtaccctgcccccatcccgggatgagctgaccaagaacca
ggtcagcctgagttgcgcggtcaaaggcttctatcccagcgacatcgccgtggagtgggagagcaatgggcagccggagaacaactacaa
gaccacgcctcccgtgttggactccgacggctccttcaagctcgtcagcaagctcaccgtggacaagagcaggtggcagcaggggaacgt
cttctcatgctccgtgatgcatgaggctctgcacaaccactacacgcagaagagcctctccctgtctccgggtaaa
SEQ ID NO.23: the amino acid sequence of human IFNα2-Fc (hole) in the anti-PDL1-IFNα (human) heterodimer (wherein the underlined bold sequence is a linker sequence) 
CDLPQTHSLGSRRTLMLLAQMRRISLFSCLKDRHDFGFPQEEFGNQFQKAETIPVLHE
MIQQIFNLFSTKDSSAAWDETLLDKFYTELYQQLNDLEACVIQGVGVTETPLMKEDSILAV
RKYFRRITLYLKEKKYSPCAWEVVRAEIMRSFSLSTNLQESLRSKEGGGGSGGGGSGGG
GSRTDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTI
SKAKGQPREPQVYTLPPCRDELTKNQVSLWCLVKGFYPSDIAVEWESNGQPENNYKTTPP
VLDSDGSFFLYSALTVDKSRWQQGNVFSCSVMHEALHNHYTQKSLSLSPGK
SEQ ID NO.24: the nucleotide sequence encoding human IFNα2-Fc (hole) in the anti-PDL1-IFNα (human) heterodimer (wherein the underlined bold sequence is a linker sequence) 
tgcgatttaccccagacacactctttaggctctcgtaggaccctcatgctgctggcccagatgaggaggatctctttattctcttgtttaaa
ggacagacacgacttcggcttcccccaagaagagttcggcaaccagttccagaaggctgagaccatccccgtgctgcacgagatgatccag
cagatcttcaatttatttagcaccaaggactccagcgccgcttgggatgagactttactggacaagttctacaccgaactgtaccagcagctgaa
cgatttagaggcttgtgtgatccaaggtgtgggagtgaccgagacccctctgatgaaggaggactccattttagccgtgaggaagtactttcgt
aggatcaccctctacctcaaggagaagaagtacagcccttgcgcttgggaggtggtgagggctgagattatgaggagcttctctttatccacc
aatctgcaagaatctttaaggtccaaggaaggcggaggcggatcaggcggcggtggatctgggggtggcggatctcgtacggacaaaa
cccacacatgcccaccttgtcccgcccctgaactcctgggcggaccgtcagtcttcctcttccccccaaaacccaaggacaccctcatgatctc
ccggacccctgaggtcacatgcgtggtggtggacgtgagccacgaagaccctgaggtcaagttcaactggtacgtggacggcgtggaggt
gcataatgccaagacaaagccgcgggaggagcagtacaacagcacgtaccgtgtggtcagcgtcctcaccgtcctgcaccaggactggct
gaatggcaaggagtacaagtgcaaggtctccaacaaagccctcccagcccccatcgagaaaaccatctccaaagccaaagggcagccccg
agaaccacaggtgtacaccctgcccccatgtcgggatgagctgaccaagaaccaggtcagcctgtggtgcctggtcaaaggcttctatccca
gcgacatcgccgtggagtgggagagcaatgggcagccggagaacaactacaagaccacgcctcccgtgttggactccgacggctccttctt
cctctacagcgcgctcaccgtggacaagagcaggtggcagcaggggaacgtcttctcatgctccgtgatgcatgaggctctgcacaaccact
acacgcagaagagcctctccctgtctccgggtaaa
SEQ ID NO.25: the amino acid sequence of human IFNα2-Fc (no ADCC) in the anti-PDL1-IFNα (human) heterodimer (wherein the underlined bold sequence is a linker sequence) 
CDLPQTHSLGSRRTLMLLAQMRRISLFSCLKDRHDFGFPQEEFGNQFQKAETIPVLHE
MIQQIFNLFSTKDSSAAWDETLLDKFYTELYQQLNDLEACVIQGVGVTETPLMKEDSILAV
RKYFRRITLYLKEKKYSPCAWEVVRAEIMRSFSLSTNLQESLRSKEGGGGSGGGGSGGG
GSRTDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEKTI
SKAKGQPREPQVYTLPPCRDELTKNQVSLWCLVKGFYPSDIAVEWESNGQPENNYKTTPP
VLDSDGSFFLYSALTVDKSRWQQGNVFSCSVMHEALHNHYTQKSLSLSPGK
SEQ ID NO.26: the nucleotide sequence encoding human IFNα2-Fc (no ADCC) in the anti-PDL1-IFNα (human) heterodimer (wherein the underlined bold sequence is a linker sequence)

tgcgatttaccccagacacactctttaggctctcgtaggaccctcatgctgctggcccagatgaggaggatctctttattctcttgtttaaa
ggacagacacgacttcggcttcccccaagaagagttcggcaaccagttccagaaggctgagaccatccccgtgctgcacgagatgatccag
cagatcttcaatttatttagcaccaaggactccagcgccgcttgggatgagactttactggacaagttctacaccgaactgtaccagcagctgaa
cgatttagaggcttgtgtgatccaaggtgtgggagtgaccgagacccctctgatgaaggaggactccattttagccgtgaggaagtactttcgt
aggatcaccctctacctcaaggagaagaagtacagcccttgcgcttgggaggtggtgagggctgagattatgaggagcttctctttatccacc
aatctgcaagaatctttaaggtccaaggaaggcggaggcggatcaggcggcggtggatctgggggtggcggatctcgtacggacaaaa
cccacacatgcccaccttgtcccgcccctgaagccgcaggcggaccgtcagtcttcctcttccccccaaaacccaaggacaccctcatgatct
cccggacccctgaggtcacatgcgtggtggtggacgtgagccacgaagaccctgaggtcaagttcaactggtacgtggacggcgtggagg
tgcataatgccaagacaaagccgcgggaggagcagtacaacagcacgtaccgtgtggtcagcgtcctcaccgtcctgcaccaggactggct
gaatggcaaggagtacaagtgcaaggtctccaacaaagccctcggagcccccatcgagaaaaccatctccaaagccaaagggcagcccc
gagaaccacaggtgtacaccctgcccccatgtcgggatgagctgaccaagaaccaggtcagcctgtggtgcctggtcaaaggcttctatccc
agcgacatcgccgtggagtgggagagcaatgggcagccggagaacaactacaagaccacgcctcccgtgttggactccgacggctccttc
ttcctctacagcgcgctcaccgtggacaagagcaggtggcagcaggggaacgtcttctcatgctccgtgatgcatgaggctctgcacaacca
ctacacgcagaagagcctctccctgtctccgggtaaa

## Claims

1. A fusion protein comprising a PDL1-binding polypeptide and an interferon (IFN) which are operably linked.

2. The fusion protein according to claim 1, which is a heterodimeric protein or a homodimeric protein.

3. The fusion protein according to claim 1, which is a heterodimeric protein and said PDL1-binding polypeptide is in the form of a Fab, wherein:
optionally, said fusion protein comprises a first polypeptide, a second polypeptide and a third polypeptide; wherein, said first polypeptide comprises an IFN and an immunoglobulin Fc region, and the IFN is located at the N-terminus or the C-terminus of the Fc region; said second polypeptide comprises a Fab region of the heavy chain of the PDL1-binding polypeptide and an immunoglobulin Fc region, and said Fab region of the heavy chain of the PDL1-binding polypeptide is located at the N-terminus or the C-terminus of the Fc region; and said third polypeptide is the light chain of the PDL1-binding polypeptide;
preferably, said first polypeptide comprises or consists of the amino acid sequence as set forth in SEQ ID NO.1 or SEQ ID NO.19 or SEQ ID NO.23 or SEQ ID NO.25, said second polypeptide comprises or consists of the amino acid sequence as set forth in SEQ ID NO.5 or SEQ ID NO.21, and said third polypeptide comprises or consists of the amino acid sequence as set forth in SEQ ID NO.3;
or, said fusion protein is a heterodimeric protein and said PDL1-binding polypeptide is in the form of a scFv, wherein:
optionally, said fusion protein comprises a first polypeptide and a second polypeptide; said first polypeptide comprises an IFN and an immunoglobulin Fc region, and the IFN is located at the N-terminus or C-terminus of the Fc region; and said second polypeptide comprises a PDL1-binding polypeptide in the form of a scFv and an immunoglobulin Fc region, and the PDL1-binding polypeptide in the form of a scFv is located at the N-terminus or C-terminus of the Fc region;
preferably, said first polypeptide comprises or consists of the amino acid sequence as set forth in SEQ ID NO. 1 or SEQ ID NO. 23, and said second polypeptide comprises or consists of the amino acid sequence as set forth in SEQ ID NO. 9.

4. The fusion protein according to claim 1, wherein said fusion protein is a homodimeric protein and said PDL1-binding polypeptide is in the form of a Fab; wherein:
optionally, said fusion protein comprises a first polypeptide and a second polypeptide which are identical, and a third polypeptide and a fourth polypeptide which are identical; wherein, said first polypeptide and second polypeptide comprise an IFN, a Fab region of the heavy chain of the PDL1-binding polypeptide, and an immunoglobulin Fc region, respectively, preferably comprise or consist of the amino acid sequence as set forth in SEQ ID NO. 7; and said third polypeptide and fourth polypeptide are respectively the light chain of the PDL1-binding polypeptide, preferably comprise or consist of the amino acid sequence as set forth in SEQ ID NO.3;
or, said fusion protein is a homodimeric protein and said PDL1-binding polypeptide is in the form of a scFv; wherein:
optionally, said fusion protein comprises a first polypeptide and a second polypeptide both of which are identical, wherein said first polypeptide and second polypeptide respectively comprise an IFN, a PDL1- binding polypeptide in the form of a scFv and an immunoglobulin Fc region, preferably comprise or consist of the amino acid sequence as set forth in SEQ ID NO. 11.

5. The fusion protein according to claim 1, wherein said interferon (IFN) can be selected from a type I interferon and a type I interferon mutant, preferably mouse IFNα4 and human IFNα2; said IFN can be of human or murine origin.

6. The fusion protein according to claim 3 or 4, wherein said immunoglobulin Fc region can be selected from the amino acid sequence of the constant region of IgG1, IgG2, IgG3 or IgG4 (preferably IgG1) or a mutant thereof .

7. The fusion protein according to claim 1, wherein, said PDL1-binding polypeptide can be selected from an anti-PDL1 antibody or an antigen-binding fragment thereof, such as a single-chain variable fragment (scFv), a Fab fragment, a F(ab')₂ fragment; and said anti-PDL1 antibody is preferably selected from: Tecentriq, Bavencio, Imfinzi, KN035, CS1001, KL-A167, SHR-1316 or YW243.55.S70; more preferably, said PDL1-binding polypeptide is a Fab fragment of a PDL1 antibody.

8. Use of the fusion protein of any one of claims 1-7 in the manufacture of a medicament for the treatment of chronic hepatitis B.

9. A pharmaceutical formulation or pharmaceutical composition comprising the fusion protein of any one of claims 1-7 as an active ingredient.

10. A polynucleotide encoding the fusion protein according to any one of claims 1-7.

11. A vector comprising the polynucleotide of claim 10, wherein said vector is optionally a plasmid vector or a viral vector.

12. A cell comprising the polynucleotide of claim 10 or the vector of claim 11, wherein, said cell expresses the fusion protein of any one of claims 1-7; preferably, said cell is selected from a non-human mammalian cell, preferably CHO and HEK293 cell.

13. A pharmaceutical composition or kit comprising the fusion protein of any one of claims 1-7 and a vaccine; preferably said fusion protein and said vaccine are administered by sequential or simultaneous administration, and the administration method comprises an intravenous injection and a subcutaneous or intramuscular immunization, respectively, or a simultaneous subcutaneous or intramuscular immunization, etc.;
optionally, the antigen in said vaccine can be an envelope protein of HBV (S-HBsAg, M-HBsAg, L-HBsAg) or a polypeptide thereof, a nucleocapsid protein (HBcAg) or a polypeptide thereof, a polymerase protein or a polypeptide thereof, a HBxAg protein or a polypeptide thereof, preferably an envelope protein of HBV or a polypeptide thereof; or the antigen in said vaccine can be a receptor-binding region antigen of SARS-CoV-2 or a HA1 antigen of influenza virus;
optionally, said vaccine further comprises an adjuvant comprising an aluminium adjuvant, a liposome, or CpG, etc.

14. Use of the fusion protein of any one of claims 1-7 or the combination of the fusion protein of any one of claims 1-7 and a vaccine in the manufacture of a medicament or kit for the prevention of a viral infection (e.g., an acute viral infection), for the treatment of a chronic or latent viral infection; optionally, said virus includes, but is not limited to, HBV, coronavirus, and influenza virus.
